(19) **Europäisches Patentamt**
European Patent Office
Office européen des brevets

(11) **EP 3 996 772 B1**

(12) **EUROPÄISCHE PATENTSCHRIFT**

(45) Veröffentlichungstag und Bekanntmachung des Hinweises auf die Patenterteilung:
**14.05.2025  Patentblatt 2025/20**

(21) Anmeldenummer: **20768275.8**

(22) Anmeldetag: **08.07.2020**

(51) Internationale Patentklassifikation (IPC):
*A61M 1/36* (2006.01)     *A61M 60/109* (2021.01)
*A61M 60/221* (2021.01)    *A61M 60/232* (2021.01)
*A61M 60/38* (2021.01)     *A61M 60/419* (2021.01)
*A61M 60/515* (2021.01)    *A61M 60/531* (2021.01)
*A61M 60/546* (2021.01)    *A61M 60/569* (2021.01)
*A61M 60/849* (2021.01)

(52) Gemeinsame Patentklassifikation (CPC):
A61M 1/3621; A61M 1/3639; A61M 1/3666;
A61M 60/109; A61M 60/221; A61M 60/232;
A61M 60/38; A61M 60/419; A61M 60/515;
A61M 60/531; A61M 60/546; A61M 60/569;
A61M 60/849; A61M 2205/3334

(86) Internationale Anmeldenummer:
**PCT/EP2020/069310**

(87) Internationale Veröffentlichungsnummer:
**WO 2021/005133 (14.01.2021 Gazette 2021/02)**

(54) **STEUERUNG FÜR NICHT-OKKLUSIVE BLUTPUMPEN**

CONTROL FOR NON-OCCLUSIVE BLOOD PUMPS

COMMANDE POUR POMPES À SANG NON OCCLUSIVES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **10.07.2019   DE 102019004825**

(43) Veröffentlichungstag der Anmeldung:
**18.05.2022   Patentblatt 2022/20**

(73) Patentinhaber: **Xenios AG**
**74076 Heilbronn (DE)**

(72) Erfinder: **SIMUNDIC, Ivo**
**73240 Wendlingen (DE)**

(74) Vertreter: **Graf von Stosch**
**Patentanwaltsgesellschaft mbH**
**Prinzregentenstraße 22**
**80538 München (DE)**

(56) Entgegenhaltungen:
US-A1- 2007 083 077     US-A1- 2008 249 456
US-A1- 2015 030 502     US-A1- 2015 141 911
US-A1- 2016 166 749

EP 3 996 772 B1

**Beschreibung**

Technisches Gebiet

Die vorliegende Erfindung betrifft Steuer- und Regeleinheiten für nicht-okklusive Blutpumpen einer extrakorporalen Kreislaufunterstützung sowie Systeme umfassend eine solche Steuer- und Regeleinheit und entsprechende Verfahren.

Stand der Technik

Wenn die Pumpenleistung oder Pumpfunktion des Herzens versagt, kann ein kardiogener Schock auftreten, was aufgrund einer Verringerung der Herzleistung bzw. des Herzausfwurfs zu einer Minderperfusion oder Durchblutung der Endorgane wie des Gehirns, der Nieren, und des Gefäßsystems im Allgemeinen führen kann. Durch dieses akute Herzversagen entsteht im Gewebe und in den Organen eine akute Blutunterversorgung und somit eine Sauerstoffunterversorgung, auch Hypoxie genannt, was zu Endorganschaden führen kann. In den meisten Fällen tritt ein solcher kardiogener Schock infolge einer Komplikation bei einem akuten Myokardinfarkt (AMI) oder Herzinfarkt auf und gehört dieser somit zu den häufigsten Todesursachen bei Myokardinfarkten. Alleine in Deutschland werden jährlich etwa 350.000 Menschen durch ein Myokard getroffen, wovon etwa zehn Prozent einen kardiogenen Schock entwickeln.

Weiterhin können kardiogene Schocks als Komplikation infolge einer chirurgischen Behandlung wie eines Bypasses oder durch eine unzureichende oder beeinträchtigte Lungenfunktion auftreten, sowie durch Störungen des Reizleitungssystems, strukturelle Herzerkrankung oder entzündliche Prozesse des Myokards entstehen. Obwohl Faktoren wie eine frühe Revaskularisierung, das Verabreichen von inotropischen Medikamenten und mechanische Unterstützung den physiologischen Zustand des Patienten verbessern können, liegt die Sterberate im Falle eines kardiogenen Schocks weiterhin über fünfzig Prozent.

Um den Zustand des Patienten zu verbessern, sind Kreislaufunterstützungssysteme entwickelt worden, welche eine mechanische Unterstützung bereitstellen und rasch mit dem Kreislauf verbunden werden können, um den Blutfluss und die Perfusion der Organe, inklusive der herzeigenen Kranzgefäße, zu verbessern und einen hypoxischen Zustand zu vermeiden. Beispielsweise sind Systeme entwickelt worden, wobei ein Ballon invasiv in den Aortenbereich eingeführt wird und derart gesteuert wird, dass dieser während der diastolischen Phase des Herzzyklus rasch aufgepumpt wird, beispielsweise mit Helium, um die Durchblutung des Herzmuskelgewebes in dieser Phase zu verbessern. Solche Systeme sind unter dem Begriff "intraaortic balloon counterpulsation" (IABP) bekannt. Die Wirkung solcher Systeme ist jedoch stark begrenzt, zumal nur Blut in die Kranzgefäße befördert wird, das während eines kardiogenen Shocks nicht hinreichend oxygeniert wurde. Daher bietet dieses Verfahren gegenüber einer medikamentösen Einstellung mit beispielsweise inotropischen Medikamenten oder Katecholaminen keine signifikante Verbesserung.

Weiterhin sind extrakorporale Kreislaufunterstützungssysteme bekannt, welche darauf basieren, dass Blut durch einen in die Femoralvene eingebrachte Kanüle aus beispielsweise dem rechten Vorhof oder der Hohlvene entnommen und über einen Membranventilator dem Patienten mittels einer in die Femoralarterie eingebrachten Perfusionskanüle wieder zugeführt wird, wobei durch den Membranventilator eine Dekarbolisierung, also Kohlendioxid-Entfernung, und Oxygenierung des Bluts erfolgt. Solche Systeme sind auch unter dem Begriff "extracorporeal membrane oxygenation" (ECMO) bekannt.

Obwohl hierdurch eine bessere Perfusion von Organen erreicht wird, erfolgt die Perfusion kontinuierlich während sowohl der systolischen als auch der diastolischen Phase, sodass durch diese Anordnung ein retrograder Blutfluss entsteht, welcher gegen die eigentliche Auswurfrichtung des Herzens gerichtet ist und somit die Nachlast erhöht. Dies hat zur Folge, dass das Herz in seinem Auswurf gehindert wird und sich in einem geringeren Maß entleert während das Herz gleichzeitig mehr Muskelarbeit verrichten muss und entsprechend eine erhöhte Sauerstoffversorgung braucht. Dies ist gerade beim ohnehin durch einen Myokardinfarkt geschädigten Herzen unvorteilhaft. Dadurch wird ein solches System vorwiegend in Fällen eines Herzstillstands oder einer beabsichtigten Umgehung der patienteneigenen Herzleistung, beispielsweise während einer chirurgischen Anwendung oder Intervention, verwendet. Zur Nachlastverringerung bei einem schlagenden Herzen kann auch vorgesehen sein, dass der Patient medikamentös durch das Verabreichen von sogenannten Vasopressoren eingestellt wird, welche die Gefäße weit stellen und somit den Widerstand und die Nachlast senken. In diesen Fällen wird eine vorhandene Herzleistung des Patienten jedoch weniger therapeutisch unterstützt, sondern es wird die Herzleistung vielmehr durch das System ersetzt.

Weiterhin erfolgt die Durchblutung der herzeigenen Koronararterien, welche den Herzmuskel im Normalfall mit ausreichend Sauerstoff versehen, im Allgemeinen in der Diastole des Herzzyklus und ist diese somit auf eine entsprechende Entleerung des linken Ventrikels angewiesen. Denn, wenn der Füllungsdruck am Ende der Systole, bzw. zu Beginn der Diastole, im linken Ventrikel so gering wie möglich ist, können die Koronararterien ihr Lumen größtmöglich entfalten, um somit die Blutflussrate und die Sauerstoffversorgung zu steigern. Durch das perkutane femoro-femoral angeschlossene extrakorporale Kreislaufsystem und den dadurch verursachten retrograden Fluss, wird die Entleerung des linken Ventrikels verringert, sodass das System für die Perfusion der Koronararterien ebenfalls unvorteilhaft ist. Zudem kann der Fluss der Blutpumpe derart eingestellt sein, dass der Blutfluss die Aortenklappen verschließt. Dadurch, dass Blut von der Pulmonalvene weiter über den rechten Vorhof in den linken Ventrikel gepumpt wird und daraus nicht entweichen kann, nimmt der Druck im linken Ventrikel in diesem Fall weiter zu, was eine weitere Verringerung der Perfusion der Koronararterien zufolge haben kann. Mit anderen Worten hat die für die Endorgane vorteilhafte unterstütze Perfusion eine beeinträchtigende Auswirkung auf die Koronarperfusion.

Ein weiteres Problem bei vielen mechanischen Unterstützungssystemen ist, dass diese keinen Blutfluss bereitstellen, welcher einem normalen physiologischen Blutfluss, d.h. einem pulsatilen Blutfluss, entspricht. Obwohl Systeme mit pulsatilen oder nicht-okklusiven Blutpumpen bekannt sind und eine verbesserte Perfusion für die Endorgane bewirken können, wurde die negative Auswirkung auf die Nachlast und die entsprechende Verringerung der Perfusion der Koronararterien bisher nicht hinreichend berücksichtigt. Durch die Höhe der Herzfrequenz, welche ebenfalls variabel sein kann, und die entsprechende kurze Dauer der jeweiligen Phasen, sind die Möglichkeiten zum Bereitstellen einer hinreichenden Perfusion der Endorgane bei einer gleichzeitigen verbesserten Perfusion der Koronararterien stark beschränkt. Um eventuelle Schwankungen zu berücksichtigen, werden allenfalls einen im Allgemeinen geringeren Blutfluss oder auch eine kürzere pulsatile Perfusion eingestellt, welche jedoch fest eingestellt ist und keine für den physiologischen Zustand optimierte Perfusion ermöglicht.

Entsprechend besteht ein Bedarf, die Eigenschaften eines Blutflusses, die durch eine Blutpumpe bereitgestellt wird, derart zu optimieren, dass diese an den physiologischen Zustand des Patienten anpassbar sind und sowohl eine Optimierung der Perfusion der Endorgane als auch der Koronararterien bereitstellt. US 2015/0141911 A1 offenbart eine Blutpumpe, die pulsatile Blutströmung in einem extrakorporalen Blutkreislaufsystem bereitstellt.

## Darstellung der Erfindung

Ausgehend von dem bekannten Stand der Technik ist es eine Aufgabe der vorliegenden Erfindung, eine verbesserte Perfusion der Koronararterien bei einer extrakorporalen Kreislaufunterstützung zu ermöglichen.

Die Aufgabe wird durch die unabhängigen Ansprüche gelöst. Vorteilhafte Weiterbildungen ergeben sich aus den Unteransprüchen, der Beschreibung und den Figuren.

Entsprechend wird eine Steuer- und Regeleinheit für eine nicht-okklusive Blutpumpe einer extrakorporalen Kreislaufunterstützung vorgeschlagen, welche dazu eingerichtet ist einen Flusswert der extrakorporalen Kreislaufunterstützung zu empfangen, eine Messung eines arteriellen Drucks und eines EKG-Signals eines unterstützten Patienten über einen vorgegebenen Zeitraum zu empfangen, einen mittleren arteriellen Druck der extrakorporalen Kreislaufunterstützung oder der unterstützten Patienten anhand der Messung des arteriellen Drucks und einen energieäquivalenten Druck anhand des Flusswerts und des arteriellen Drucks zu bestimmen, und einen erforderlichen Wert von mindestens einem Pulsparameter der Blutpumpe anhand des mittleren arteriellen Drucks, des energieäquivalenten Drucks und des EKG-Signals zu bestimmen, um ein Verhältnis des energieäquivalenten Drucks zum mittleren arteriellen Druck zu bewirken, welches größer als 1,0 ist. Die Steuer- und Regeleinheit ist weiterhin dazu eingerichtet ist, den Pulsparameter in Abhängigkeit des EKG-Signals einzustellen.

Die Steuer- und Regeleinheit kann beispielsweise eine oder mehrere in einem System zur extrakorporalen Kreislaufunterstützung vorhandenen Pumpenantriebe oder Pumpenköpfe für nicht-okklusive Blutpumpen steuern bzw. regeln. Die Blutpumpe kann zur Sauerstoffanreicherung und Dekarbolisierung, also Kohlendioxid($CO_2$)-Entfernung bzw. - Abreicherung, des Bluts angeordnet sein. Beispielsweise kann die Blutpumpe mit einem im extrakorporalen Kreislaufunterstützungssystem vorgesehenen Oxygenator oder Membranventilator fluidisch verbunden sein, welcher beispielsweise mit einem venösen Zugang mittels einer venösen Kanüle und einem arteriellen Zugang mittels einer arteriellen Kanüle zum Ansaugen bzw. Fördern des Bluts verbunden ist, um einen Blutfluss von einer Seite mit einem niedrigen Druck zu einer Seite mit einem höheren Druck bereitzustellen. Derart können mittels der Blutpumpe eine extrakorporale Sauerstoffanreicherung des Bluts und eine verbesserte Sauerstoffversorgung für den Patienten bereitgestellt werden. Diese Merkmale können aufgrund des pulsatilen Charakters der nicht-okklusiven Blutpumpe sowie des Verhältnisses zwischen dem energieäquivalenten Druck und dem mittleren arteriellen Druck weiter verbessert werden, insbesondere im Hinblick auf die Perfusion der Koronararterien und der Endorgane. Durch Vorgabe des vorgenannten Verhältnisses ist die Steuer- und Regeleinheit nicht abhängig von festen oder vorgegebenen Werten des Pulsparameters. Insbesondere kann der Pulsparameter unmittelbar an die physiologischen Erfordernisse des jeweiligen Patienten angepasst und optimiert werden.

Die nicht-okklusive Blutpumpe kann beispielsweise magnetisch mit dem Pumpenantrieb eines Motors gekoppelt sein, um eine Drehmomentübertragung zu ermöglichen. Dabei kann als nicht-okklusive Blutpumpe eine Kreiselpumpe wie eine Rotorpumpe bzw. Impellerpumpe vorgesehen sein, beispielsweise eine Zentrifugalpumpe oder eine Halbaxial-Pumpe, auch bekannt als Diagonalpumpe. Während mit einer Zentrifugalpumpe ein hoher Druck bei einer geringen Flussrate erzeugt werden kann, können Diagonalpumpen eine hohe Flussrate bei einem geringen Druck erzeugen, was den allgemeinen Voraussetzungen für pulsatile Blutpumpsysteme entspricht und insbesondere für eine extrakorporale Kreislaufunterstützung und respiratorische Unterstützung bei einem kardiogenen Schock vorteilhaft ist. Beispielsweise kann durch die rasche Bereitstellung einer hohen Flussrate mit einer geringen Druckhöhe eine verbesserte und genauere Synchronisierung anhand eines EKG-Signals erfolgen.

Weiterhin kann eine Rotorpumpe im Gegensatz zu einer Rollerpumpe oder okklusive Blutpumpe zwar keinen Druck aufbauen und diesen zu einem vorgegebenen Zeitpunkt loslassen. Durch Änderung der Drehzahl des Rotorblattes bzw. des Impellers lässt sich jedoch ein geringer Druck aufbauen, welcher als Fluss weitergeleitet wird und den Vorteil hat, dass ein genaues Pulsieren ermöglicht wird, ohne dabei weitere Komponenten wie Ventile zu benötigen. Weiterhin können durch eine geringe Dimensionierung des Rotorblattes / Impellers ebenfalls eine geringe Trägheit und somit eine sehr genaue Ansteuerung und präzise Abgabe eines Pulses bei jeder Drehzahl erreicht werden.

Dadurch, dass menschliche Organe und Zellen von Natur aus dynamische Fluss- und Druckänderungen detektieren und verarbeiten, reagieren diese ebenfalls unterschiedlich auf unterschiedliche Flussprofile. Insbesondere findet im vaskulären Endothel, welches die innerste Schicht der Gefäße bildet, in Reaktion auf Schubspannung und Pulsdruck eine Mechanotransduktion und Signalübertragung statt, welche Funktionen wie Apoptose, Angiogenese, Atherosklerose, und den systemischen Blutdruck regeln. In dieser Hinsicht ist die nicht-okklusive Blutpumpe weiter vorteilhaft, zumal diese einen pulsatilen Blutfluss bereitstellt, wodurch die mechanische und/oder thermische Belastung von Blutzellen reduziert und insbesondere die Zerstörung von roten Blutzellen und die Aktivierung von Blutplättchen verhindert werden.

Im Allgemeinen bewirkt der pulsatile Blutfluss weiterhin eine verbesserte Mikrozirkulation der vitalen Organe, eine verbesserte vaskuläre Kompliance, eine Verringerung der inotropischen Unterstützung, eine höhere Sauerstoffsättigung des Gehirns, eine erhöhte Harnausscheidung und eine Verringerung von gastrointestinalen Blutungsraten, wodurch Patienten generell eine kürzere Zeit im Krankenhaus verbringen müssen.

Die Blutpumpe kann mit einem venösen Zugang mittels einer venösen Kanüle und einem arteriellen Zugang mittels einer arteriellen Kanüle zum Ansaugen bzw. Fördern des Bluts verbunden sein, um einen Blutfluss von einer Seite mit einem niedrigen Druck zu einer Seite mit einem höheren Druck bereitzustellen. Bevorzugt ist die Blutpumpe als Disposable oder Einmalartikel ausgebildet und fluidisch von dem jeweiligen Pumpenantrieb getrennt und leicht koppelbar, beispielsweise über eine magnetische Kopplung. Die Steuer- und Regeleinheit betätigt dabei den Motor des Pumpenantriebs und ist dazu eingerichtet, die Drehzahl des Rotorblatts der Blutpumpe derart einzustellen, dass die Blutpumpe entsprechend einen wellenartigen Fluss erzeugt, welcher einem pulsatilen Blutfluss entspricht.

Weiterhin kann durch die Änderung der Drehzahl die Pulsamplitude vorgegeben werden, welche an der Steuer- und Regeleinheit eingegeben und optional auf einen Grundfluss aufgerechnet werden kann. Beispielsweise kann ein Grundfluss vorgegeben sein, wobei die jeweiligen Pulse eine Erhöhung des gesamten Blutflusses bewirken. Daraus ergibt sich ein Mittelfluss, welche entsprechend durch die Steuer- und Regeleinheit berechnet wird. Beispielsweise kann die Blutpumpe für eine Drehzahl zwischen etwa 50 RPM und etwa 10.000 RPM eingerichtet sein, um einen Blutfluss zwischen etwa 0,1 l/min und etwa 8 l/min bereitzustellen, wobei sich der pulsatile Fluss aus der Drehzahländerung ergibt. Bevorzugt wird jedoch ein Blutfluss zwischen etwa 0,5 l/min und etwa 5,0 l/min (beispielsweise zwischen 1 l/min und 2,5l /min), um einerseits eine Gerinnung und andererseits eine Nachlasterhöhung zu verhindern, und dabei einen gewünschten Fluss zu erreichen, der eine adäquate Organ-Oxygenierung, sprich eine ausreichende Anzahl an sauerstoffbeladener roter Blutkörperchen, bereitstellt.

Ein Vorteil des pulsatilen Blutflusses liegt weiterhin darin, dass eine Pulsenergie erzeugt wird, welche sich aus der Druckänderung im Hinblick auf den Flussgradienten über Zeit ergibt. So ergibt sich aus der Multiplikation der jeweiligen Integrale die hämodynamische Arbeit, welche geteilt durch das Flussintegral den energieäquivalenten Druck ergibt, auch bekannt als "energy equivalent pressure" (EEP). Dieses Verhältnis ist mathematisch in der folgenden Formel wiedergegebenen:

$$\mathrm{EEP\,(mm\,Hg)} = \int_{t1}^{t2} fpdt \Big/ \int_{t1}^{t2} fdt$$

wobei der Blutfluss (f) und der Druck (p) über ein Zeitintervall von t1 bis t2 berücksichtigt werden.

Der energieäquivalente Druck ist vergleichbar mit dem Blutdruck des Patienten und basiert auf der Idee, dass hämodynamische Energie ausgehend von der Aortenwurzel durch die axiale Bewegung des Blutflusses und die elastische Bewegung der Aortenwand an die Peripherie übertragen wird. Entsprechend kann der energieäquivalente Druck weiterhin in eine überschüssige hämodynamische Energie, auch bekannt als "surplus hemodynamic energy" (SHE) umgerechnet werden, welche bevorzugt die Energie für einen einzelnen Puls wiedergibt und der vom pulsatilen Fluss im Hinblick auf den nicht-pulsatilen Fluss oder Grundfluss erzeugten zusätzlichen Energie entspricht. Somit stellen sowohl der energieäquivalente Druck als auch die überschüssige hämodynamische Energie quantifizierbare Maße für die Unterstützung des Kreislaufs und/oder des Herzens bereit.

Um eine Verbesserung gegenüber einem konstanten Blutfluss und eine Erhöhung des Blutdrucks des Patienten zu bewirken, sollte der energieäquivalente Druck höher als der mittlere arterielle Druck sein, der auch als "mean arterial pressure" (MAP) bekannt ist. Mit anderen Worten bewirkt ein Verhältnis des energieäquivalenten Drucks zum mittleren arteriellen Druck, welches mindestens 1,0 oder größer als 1,0 (beispielsweise > 1,0 und < 1,25) ist, dass ein physiologischer Blutfluss bereitgestellt wird. Es wird also ein Pulsbeitrag berechnet, welcher eine Erhöhung des mittleren arteriellen Drucks bewirkt. Beispielsweise kann der Pulsbeitrag minimal 5 mmHg betragen und bevorzugt zwischen etwa 10 mmHg und 20 mmHg liegen. Das durch die Steuer- und Regeleinheit bereitgestellte Pulsen ist somit als physiologisches oder quasi-physiologisches Pulsen zu verstehen.

Beispielsweise kann der energieäquivalente Druck durch den pulsatilen Blutfluss bzw. durch das Pulsen der Blutpumpe selbst gegenüber einem konstanten Blutfluss beim selben mittleren arteriellen Druck höher sein, sodass eine verbesserte Durchblutung der distalen Gefäße bereitgestellt wird. Der Unterschied im energieäquivalenten Druck ist dabei ein Indikator für den benötigten höheren mittleren Druck bei einem nicht-pulsatilen Fluss, um dieselbe Durchblutung zu bewirken.

Entsprechend kann der energieäquivalente Druck durch die Steuer- und Regeleinheit bestimmt werden, wobei der Flusswert, der durch die Blutpumpe bereitgestellt wird, manuell eingegeben werden kann, beispielsweise über die Drehzahl des Rotorblatts, und lediglich eine Messung des arteriellen Drucks erforderlich ist. Aus dieser Messung kann ebenfalls der mittlere arterielle Druck durch Mittelung der Werte bestimmt werden.

Der arterielle Druck wird bevorzugt über eine Schnittstelle, welche kommunikativ mit mindestens einem Drucksensor verbunden ist, in die Steuer- und Regeleinheit eingespeist. Bevorzugt erfolgt die Druckmessung vor der Blutpumpe, hinter der Blutpumpe und/oder nach einem im extrakorporalen Kreislaufsystem vorgesehenen und mit der Blutpumpe verbundenen Oxygenator bzw. Membranventilator. Die Druckmessung eines arteriellen Drucks des unterstützten Patienten erfolgt bevorzugt invasiv im Patienten, beispielsweise an einer Kanülenspitze einer arteriellen Kanüle, beispielsweise in der Aorta des Patienten, oder durch eine andere Druckmesssonde, die in der Aorta an jeder beliebter Stelle positioniert werden kann. Alternativ kann die Messung jedoch auch an der *a. femoralis* und/oder *a. iliaca* oder oder *a. radialis* erfolgen. Darüber hinaus gibt es eine Möglichkeit, dass die Druckmessung über nicht invasive Drucksensoren (ohne direkten Blutkontakt) oder invasive Druckmessung mit Blutkontakt an den Schlauchsenkeln des Patientenkits geschehen kann.

Sowohl der Flusswert als auch der arterielle Druck auf nicht-invasive Weise können derart erfasst und von der Steuer- und Regeleinheit empfangen werden, indem beispielsweise der Flusswert über die Drehzahl des Rotorblatts und/oder der arterielle Druck über nicht-invasive Schlauschenkel oder im Kreislaufsystem außerhalb des Patienten erfasst werden. Dadurch kann das Kreislaufsystem kompakt ausgestaltet und dessen Komplexität verringert werden. Für den Patienten bedeutet dies weiterhin, dass eingeführte Kanülen kleiner dimensioniert sein können und chirurgische Eingriffe grundsätzlich weniger invasiv erfolgen können.

Aus dem bestimmten energieäquivalenten Druck und dem bestimmten mittleren arteriellen Druck kann die Steuer- und Regeleinheit das entsprechende Verhältnis bestimmen und den Pulsparameter der Blutpumpe entsprechend einstellen, um das gewünschte Verhältnis von mehr als 1,0 zu erreichen. Dies kann iterativ und bevorzugt kontinuierlich erfolgen, sodass die Auswirkung der Änderung des Pulsparameters der Blutpumpe auf den physiologischen Zustand des Patienten berücksichtigt wird und das Verhältnis zwischen dem energieäquivalenten Druck und dem mittleren arteriellen Druck nicht abhängig von festen Werten des Pulsparameters ist und optimiert werden kann.

Bevorzugt ist das Verhältnis größer als 1,1, um eine weiter verbesserte therapeutische Unterstützung des Patienten zu bewirken. Beispielsweise kann ein ermitteltes Verhältnis von 1,0 eine Erhöhung des energieäquivalenten Drucks und/oder eine Verringerung des mittleren arteriellen Drucks erfordern und kann eine erste Änderung eines Pulsparameters ein Verhältnis von 1,05 bewirken und eine zweite Änderung des Werts des Pulsparameters ein Verhältnis von 1,15 bewirken, sodass keine weitere Änderung des Pulsparameters erforderlich ist. Das Verhältnis kann weiterhin 1,15 bis 1,25, beispielsweise etwa 1,2 betragen, beispielsweise im Falle eines Herzstillstands während einer Reanimation des Patienten, wenn der mittlere arterielle Druck des Patienten in einem unteren Grenzbereich liegt.

Der Wert des Pulsparameters wird weiterhin anhand eines EKG-Signals, vorzugsweise der Herzfrequenz, eingestellt. Das EKG-Signal ist bevorzugt ein charakteristisches EKG-Signal, das eine Synchronisierung der Steuer- und Regeleinheit mit der Blutpumpe ermöglicht, sodass eine regelmäßige bzw. periodische Ausgabe eines dem Wert des Pulsparameters entsprechenden Signals von der Steuer- und Regeleinheit an den Pumpenantrieb bzw. an die Blutpumpe erfolgen kann. Beispielsweise kann das EKG-Signal bzw. der jeweilige Bereich in der elektrischen Erregungsleitung kennzeichnend für die systolische oder diastolische Phase des Herzens sein, sodass das Einstellen des Pulsparameters und das Betätigen des Motors an einem vorgegebenen Zeitpunkt und in einer vorgegebenen Phase erfolgen kann und keine Überlappung mit anderen Phasen bewirkt.

Durch die Synchronisierung des Pulses mit dem EKG-Signal wird somit ein pulsatiler Blutfluss bereitgestellt, welcher mit dem Herzzyklus des Patienten übereinstimmt. Dadurch kann die Pulsfrequenz der Blutpumpe an die Herzfrequenz und ggf. andere physiologische Parameter des Patienten angepasst werden, sodass, beispielsweise im Falle einer Tachykardie, ein Pulsparameter derart geändert werden kann, dass der Puls nicht mit einer Phase des Herzzyklus, worin der Puls nicht vorgesehen ist, überlappt oder interferiert. Es wird durch das erforderliche Verhältnis zwischen dem energieäquivalenten Druck und dem mittleren arteriellen Druck dennoch eine hinreichende Perfusion der Koronararterien und der Endorgane, im Rahmen der physiologischen Grenzen und der Pumpeneigenschaften, sichergestellt.

Gegenüber bekannten ECMO-Systemen mit einem kontinuierlichen Blutfluss können somit erfindungsgemäß sowohl ein physiologischer Blutfluss als auch eine Anpassung an den Herzzyklus des Patienten erreicht werden, und dabei die Nachlast, d.h. ein verbleibender Druck im Aortenbereich vor bzw. während der systolischen Phase, so gering wie möglich gehalten oder nicht erhöht werden.

Weiterhin werden durch den pulsatilen Fluss gegenüber einem konstanten bzw. kontinuierlichen Fluss ebenfalls ein höherer mittlerer arterieller Druck und ein höherer energieäquivalenter Druck sowie eine verbesserte überschüssige hämodynamische Energie bereitgestellt. Dadurch wird ein höherer systemischer Blutdruck bei einer geringeren inotropischen Unterstützung erreicht. Dadurch wird nicht nur eine verbesserte Perfusion der Koronararterien und der Endorgane bewirkt, sondern es kann ebenfalls eine verbesserte Sauerstoffversorgung bereitgestellt werden, beispielsweise, wenn im extrakorporalen Kreislaufunterstützungssystem ein Oxygenator oder Membranventilator vorgesehen ist. Gegenüber bekannten IABP-Systemen, welche keine oder ggf. eine unzureichende extrakorporale Kreislaufunterstützung ermöglichen, wird dadurch eine verbesserte Entlastung und Erholung des Herzmuskelgewebes erreicht.

Der mindestens ein Pulsparameter umfasst bevorzugt eine Pulsamplitude, eine Pumpendrehzahl, eine Pumpendrehzahländerung pro Zeiteinheit, eine Pulsdauer, eine systolische Pumpendauer, eine diastolische Pumpendauer, eine Antriebsradentschleunigung, eine Antriebsradbeschleunigung, einen gemittelten Pumpenfluss, einen Basisfluss und/oder einen Spitzenfluss.

Beispielsweise können durch die Pulsamplitude und die Pumpendrehzahl der maximale Blutfluss während des Pulses und somit der Mittelfluss vorgegebenen werden. Dabei wird die Pumpendrehzahl bevorzugt anhand des ermittelten Verhältnisses zwischen dem energieäquivalenten Druck und dem mittleren arteriellen Druck auf einen maximalen Wert optimiert, um eine erforderliche Pulsamplitude zu erhalten. Beispielsweise kann eine Pulsamplitude durch eine Änderung der Pumpendrehzahl bereitgestellt werden, wobei die Änderung der Pumpendrehzahl einen Wert zwischen 50 U/min und 10.000 U/min aufweisen kann und bevorzugt zwischen 1000 U/min und 5000 U/min, besonders bevorzugt zwischen 3000 U/min und 4000 U/min liegt. Somit kann ein pulsatiler Blutfluss mit einem hinreichenden Mittelfluss bereitgestellt werden, beispielsweise zwischen etwa 2 und 3 l/min. Eine Pumpendrehzahländerung kann dabei weiterhin optional auf einen Grundfluss aufgerechnet werden, wobei die erreichte maximale Pumpendrehzahl von einer Basisdrehzahl, die Pumpendrehzahländerung pro Zeiteinheit und die Pulsdauer vorgegeben ist.

Der Puls kann weiterhin durch die Pulsdauer vorgegebenen werden, wobei eine Pulsdauer bevorzugt zwischen 20 ms und 400 ms liegt. Beispielsweise kann die Pulsdauer etwa 200 ms sein, um einen hinreichenden Puls bei einer normalen Herzfrequenz bereitzustellen. Der Puls umfasst dabei eine systolische Pumpendauer und eine diastolische Pumpendauer worin eine Antriebsradbeschleunigung bzw. eine Antriebsradentschleunigung stattfinden. Diese Phasen der Pulsdauer können symmetrisch sein, sodass der systolischen Phase etwa 50% der Pulsdauer umfasst und beispielsweise 100 ms umfasst.

Die systolische Phase kann jedoch auch an die Pulsdauer angepasst sein, beispielsweise wenn die Pulsdauer aufgrund einer erhöhten Herzfrequenz verkürzt wird und die systolische Phase verlängert wird, um einen hinreichenden Puls auch bei einer verkürzten Pulsdauer bereitzustellen. In diesem Fall ist die Pulsdauer asymmetrisch, wobei die systolische Phase beispielsweise etwa 60% oder etwa 70% der Pulsdauer umfassen kann. Um zu verhindern, dass ein erhöhter Blutfluss auch nach der Pulsdauer bereitgestellt wird, kann weiterhin die Antriebsradentschleunigung während der diastolischen Phase erhöht werden. Umgekehrt kann die Antriebsradbeschleunigung auch während der systolischen Phase erhöht werden, um eine kürzere systolische Phase bei einer hinreichenden Drehzahländerung bereitzustellen. In diesem Fall kann die systolische Phase beispielsweise etwa 30% oder etwa 40% der Pulsdauer umfassen.

Entsprechend kann die Blutpumpe auch bei einem kürzeren Zeitintervall derart geregelt werden, dass eine hinreichende Perfusion bereitgestellt wird, ohne dass eine unerwünschte Überlappung des Pulses mit einer spezifischen Phase des Herzzyklus erfolgt. Dadurch kann eine unerwünschte Nachlast reduziert oder sogar verhindert werden.

Um die Pulsdauer und die Pulsenergie weiter zu verbessern, kann die während des Pulses erreichte Drehzahl passiv entschleunigt werden (ohne aktive Bremstätigkeit) oder auch aktiv gebremst werden. Im Fall aktiver Bremsung kann beispielsweise eine Vierquadrantensteuerung vorgesehen sein, wobei vier Transistoren spiegelsymmetrisch angeordnet sind und eine H-Brücke mit einem p-Kanal und einem n-Kanal bilden. Eine entsprechende Aktivierung der Transistoren ermöglicht eine Umkehrung der Stromrichtung durch einen bürstenlosen DC-Motor, sodass der Motor des Pumpenantriebs in zwei entgegengesetzte Richtungen geschaltet und eine Drehzahlreduzierung entsprechend aktiv durch eine Umkehrung der Stromrichtung erreicht werden kann.

Weiterhin kann die Blutpumpe auch derart gesteuert werden, dass ein negativer Blutfluss am Ende des Pulses bewirkt wird. Dadurch findet ein Ansaugen statt, welches beispielsweise so synchronisiert sein kann, dass dies während der systolischen Phase erfolgt, um somit die Nachlast weiter zu senken.

Als weitere Leitgröße für den Puls kann ebenfalls das Herzauswurfvolumen vorgesehen sein, das mittels einer Impedanzmessung bestimmt werden kann.

Beispielsweise kann die Impedanz über im extrakorporalen Kreislaufunterstützungssystem vorgesehene Drucksensoren, wie integrierte Drucksensoren, oder über eine Impedanzmessung im Thoraxbereich des Patienten, entweder mit transkutanen Sensoren oder eine in den Ösophagus gelegten Sonde zur Impedanzmessung, bestimmt werden.

Der erforderliche Wert des Pulsparameters kann weiterhin anhand eines vorgegebenen mittleren arteriellen Drucks und/oder eines vorgegebenen energieäquivalenten Drucks bestimmt werden. Alternativ, oder zusätzlich, kann dies anhand eines gemessenen Herzauswurfvolumens, des Schlagvolumens und/oder der Auswurffraktion bestimmt werden.

So können beispielsweise vorgegebene Schwellenwerte in der Steuer- und Regeleinheit hinterlegt sein, welche wesentlichen Werten von Vitalparameter bzw. des physiologischen Zustands des Patienten entsprechen. Mit anderen Worten können somit physiologische Grenzen für den energieäquivalenten Druck und den mittleren arteriellen Druck vorgegebenen sein, welche absolute und/oder patientenspezifische Werte sein können. So kann der mittlere arterielle Druck beispielsweise durch eine verabreichte Katecholamin-Dosis und/oder das Körpervolumen bzw. Gewicht des unterstützten Patienten vorgegeben sein. Weiterhin kann beispielsweise eine hohe Herzfrequenz des unterstützten Patienten eine Reduzierung des mittleren arteriellen Drucks erfordern, sodass der Pulsparameter auf einen entsprechenden Höchstwert des energieäquivalenten Drucks beschränkt ist. Diese Werte können weiterhin aktiv durch aktuelle Messungen von Vitalparametern vorgegeben werden, beispielsweise über eine Schnittstelle mit einem kommunikativ verbundenen EKG-Gerät.

Um die für den energieäquivalenten Druck erforderlichen Flusswert zu empfangen, kann die Steuer- und Regeleinheit weiter dazu eingerichtet sein, den Flusswert anhand einer eingegebenen Drehzahl der Blutpumpe oder automatisch anhand einer empfangenen Flussmessung zu bestimmen.

Der Pumpenflusswert kann beispielsweise einer spezifischen Drehzahl entsprechen, sodass die Eingabe der Drehzahl über eine Benutzerschnittstelle der Steuer- und Regeleinheit den Pumpenflusswert vorgibt. Bevorzugt wird der Pumpenflusswert jedoch durch einen Flusssensor gemessen, sodass zur Bestimmung des energieäquivalenten Drucks eine aktuell gemessene Strömungsgeschwindigkeit durch die Steuer- und Regeleinheit empfangen wird. Da der Flusswert weiterhin für jeden Patienten bei einer vorgegebenen Drehzahl unterschiedlich sein kann, kann der Flusssensor beispielsweise an einer Kanülenspitze der arteriellen Kanüle, der venösen Kanüle und/oder in der Leitung zwischen der Blutpumpe und der jeweiligen Spitze angeordnet sein, sodass eine genaue Messung des Flusswerts ermöglicht wird.

Bevorzugt umfasst der Flusswert einen patientenspezifischen Mittelfluss, wobei die Steuer- und Regeleinheit weiter dazu eingerichtet sein kann, den Mittelfluss anhand einer empfangenen Flussmessung und des mittleren arteriellen Drucks durch Einstellen des Pulsparameters einzustellen.

Wie oben bereits beschrieben, kann der Mittelfluss beispielsweise durch die Drehzahlerhöhung und die entsprechende Pulsamplitude bereitgestellt werden. Da der physiologische und ebenfalls der pathophysiologische Zustand eines jeden Patienten jedoch unterschiedlich ist, kann für den jeweiligen Patienten ein patientenspezifischer Mittelfluss erforderlich sein. Neben der Flussmessung, welche wie oben beschrieben durch einen im extrakorporalen Kreislaufunterstützungssystem angeordneten Flusssensor bereitgestellt werden kann, kann der für den Patienten optimale Mittelfluss anhand des mittleren arteriellen Drucks bestimmt werden und somit durch Einstellen des Pulsparameters, beispielsweise über die Drehzahl der Blutpumpe, eingestellt werden.

Dadurch wird eine automatisierte Einstellung der Blutpumpe und ebenfalls der extrakorporalen Kreislaufunterstützung ermöglicht. Neben dem mittleren arteriellen Druck und der Flussmessung können weitere Vitalparameter bei der Bestimmung des optimalen Mittelflusses berücksichtigt werden, beispielsweise die Herzfrequenz, das Alter, das Krankheitsbild und sonstige physiologische Werte des Patienten.

Die Steuerung des Fluss-, Druck- und Pulscharakteristik-Parameters kann auch über die Vorgabe eines EEP-Wertes erfolgen. Der aktuelle EEP-Wert wird im Display der Konsole angezeigt, der dann auf einen Ziel-EEP verändert werden kann. Die Anzeige kann auch als EEP-Prozent Wert erfolgen, der sich auf dem maximal möglichen EEP-Wert bei dem individuellen Patienten bezieht. Dieser Maximal-EEP-Wert kann patientenindividuell durch einen kurzen empirischen Algorithmus ermittelt werden.

Um eine optimale Synchronisierung des Pulses mit den jeweiligen Phasen des Herzzyklus zu ermöglichen, ist die Steuer- und Regeleinheit bevorzugt dazu eingerichtet, aus dem EKG-Signal eine Herzfrequenz zu bestimmen und den Pulsparameter anhand der Herzfrequenz einzustellen. Beispielsweise kann somit die Pulsdauer des Pulses an die Herzfrequenz angepasst werden und der Motor bzw. das Rotorblatt kann in dem Moment betätigt werden, wo es nicht mit einer Phase des Herzzyklus kollidiert.

Die Herzfrequenz kann alternativ ebenfalls aus einer Druckmessung, einer Druckkurve, einer Druckänderung, einem Herzauswurfvolumen, einer Auswurffraktion, und/oder einer Impedanzmessung bestimmt werden. Der Pulsparameter bzw. das Einstellen des Pulsparameters kann entsprechend an die Herzfrequenz des Patienten angepasst werden, beispielsweise durch eine Änderung der Pulsdauer und eine Anpassung der Drehzahländerung.

Der Pulsparameter kann optional ebenfalls anhand einer entsprechenden Pulsfrequenz eingestellt werden. Beispielsweise kann der Pulsparameter alternativ nur für jeden zweiten oder dritten Herzschlag eingestellt werden, sodass der Puls anstatt eines Verhältnisses von 1:1 auch in einem Verhältnis zur Herzfrequenz von 1:2 bzw. 1:3 eingestellt werden kann, wenn die Herzfrequenz eine Grenzfrequenz überschreitet. Dabei kann die Herzfrequenz für einen vorgegebenen Zeitraum überwacht oder evaluiert werden, wobei beispielsweise eine kurzfristige Erhöhung der Herzfrequenz keine Änderung der Pulsfrequenz bewirkt und/oder eine zunächst reduzierte Pulsfrequenz nach einer Normalisierung der Herzfrequenz wieder zurückgestellt bzw. erhöht werden kann.

Insbesondere kann die Steuer- und Regeleinheit dazu eingerichtet sein, aus dem EKG-Signal eine Amplitudenänderung zu bestimmen und den Pulsparameter an einem vorgegebenen Zeitpunkt nach der Amplitudenänderung einzustellen.

Beispielsweise kann als Triggersignal eine P-Welle, eine oder mehrere charakteristische Merkmale der P-Welle, ein Punkt oder Streckenabschnitt, oder charakteristische Merkmale des QRS-Komplexes bestimmt werden und der Pulsparameter an einem vorgegebenen Zeitpunkt nach der P-Welle, dem QRS-Komplex oder der R-Zacke eingestellt werden. Es können jedoch auch andere Amplitudenänderungen bestimmt werden, beispielsweise über einen vorgegebenen Streckenabschnitt des EKG-Signals oder aus einem markanten Punkt des EKG-Signals. Bevorzugt wird aus dem EKG-Signal eine R-Zacke bestimmt. Der Pulsparameter kann an einem vorgegebenen Zeitpunkt nach der Detektion der R-Zacke, beispielsweise der Detektion der maximalen Amplitude, eingestellt werden, typischerweise mit einer Verzögerung. Weiterhin kann eine Mittelung der Amplitudenänderung über einen vorgegebenen Zeitraum erfolgen, sodass beim Einstellen des Pulsparameters eventuelle Abweichungen oder Variationen berücksichtigt werden können.

Die R-Zacke kann beispielsweise durch aus einem Oberflächen-EKG des Patienten bestimmt werden, kann alternativ aber auch invasiv erfolgen, beispielsweise über eine Ableitung an der Kanülenspitze einer venösen Drainagekanüle im rechten Vorhof oder der *vena cava superior,* an der Kanülenspitze der arteriellen Kanüle in der Aorta oder der *a. femoralis* oder der *a. iliaca.* Weiterhin kann das EKG-Signal über eine invasive Ösophagus-Sonde oder über nichtinvasive Drucksensoren an Schlauchschenkeln, beispielsweise in das Schlauchsystem integrierte Drucksensoren (IPS), abgeleitet werden.

Die R-Zacke des aus dem EKG-Signal ausgelesenen QRS-Komplexes kann dann als Triggersignal verwendet werden, wobei das Pumpensignal bzw. das Einstellen des Pulsparameters nach einer vorgegebenen Verzögerung erfolgt. Beispielsweise kann die Verzögerung so gewählt sein, dass das Pumpensignal in dem Zeitpunkt erfolgt, wo eine kurze Druckerhöhung in der Aorta stattfindet, was den Zeitpunkt des Aortenklappenschlusses und somit den Anfang der Diastole markiert (Dikrot'scher Punkt). Durch Betätigung des Motors bzw. des Pumpenantriebs und somit des Rotorblatts und das Triggern des Pulses zu diesem Zeitpunkt kann ein pulsatiler Blutfluss über eine arterielle Kanüle in den Bereich (direkt) stromabwärts des linken Ventrikels und in die Aorta abgegeben werden. Die Positionierung und gegenströmige Ausrichtung der Kanülenspitze bewirken dadurch einen erhöhten pulsatilen Blutfluss in den Koronararterien und stellen eine entsprechende verbesserte Durchblutung des Herzmuskelgewebes bereit, ohne jedoch den systemischen Kreislauf zu beeinträchtigen.

Die durch den Puls bewirkte Druckerhöhung muss jedoch zum Anfang der Systole abgeschlossen sein, um eine Überlappung zu verhindern. Entsprechend ist die Pulsdauer beschränkt, was jedoch bei einem normofrequenten Herzrhythmus unproblematisch ist. Beispielsweise können zwischen der detektierten R-Zacke und dem Schließen der Aortenklappen etwa 300 ms liegen und kann eine Wartezeit zwischen der Erkennung der R-Zacke und der Abgabe des Pumpensignals ca. 200 ms, beispielsweise zwischen 150 ms und 250 ms, umfassen, sodass die Betätigung durch Ausgabe des Pumpensignals bzw. des Pulsparameters 100 ms umfassen kann. Obwohl das Pumpensignal bereits vor dem Schließen der Aortenklappen erfolgt, wird das Pumpensignal bevorzugt derart ausgegebenen, dass der Puls bzw. die Druckhöhe sich an einer Kanülenspitze befindet, wenn die Aortenklappen geschlossen sind, sodass im Hinblick auf die verwendeten Pumpe und Leitungen eine Latenzzeit für die fluidische Transportzeit berücksichtigt werden kann.

Die Pulsdauer kann dabei etwa 200 ms, beispielsweise 100 ms bis 300 ms, umfassen, wobei die systolische Phase des Pulses etwa 100 ms umfasst, wenn der Puls symmetrisch abgegeben wird. Um eine hinreichende Perfusion zu bewirken, kann dabei eine Pulsamplitude durch Einstellen einer Drehzahländerung zwischen 2500 U/min und 4500 U/min, bevorzugt etwa 3500 U/min gewählt werden.

Bei einer erhöhten Herzfrequenz, beispielsweise von 90 bis 130 Schlägen pro Minute, kann bei diesen Werten jedoch eine Flusskollision zwischen dem Pumpenauslauf und dem Systolenbeginn bzw. des Herzauswurfs auftreten. Um dies zu verhindern und die Nachlast zu optimieren, kann eine Pulsamplitude durch Einstellen einer Drehzahländerung zwischen 1500 U/min und 5000 U/min gewählt werden und kann die Pulsdauer beispielsweise zwischen 120 ms und 300 ms umfassen. Der Puls kann dabei asymmetrisch sein, wobei die systolische Phase etwa 70% bis etwa 90% umfassen kann. Somit kann auch bei einer höheren Herzfrequenz eine Nachlastverringerung und eine hinreichende Perfusion der Koronararterien bereitgestellt werden.

Entsprechend kann durch den pulsatilen Blutfluss und die Einstellung des Pulsparameters anhand der R-Zacke des QRS-Signals eine verbesserte Durchblutung sowohl der Herzmuskulatur als auch der Endorgane bereitgestellt werden, wobei die Blutpumpe und die Pulsparameter durch die entsprechende Konfiguration der Steuer- und Regeleinheit komplett an den intrinsischen Herzrhythmus des Patienten angepasst werden können. Insbesondere kann dabei durch die Pulsapplikation eine diastolische Augmentation bewirkt werden, ohne jedoch die Nachlast dabei zu sehr zu erhöhen und diese so gering wie möglich zu halten.

Bevorzugt wird eine Diagonalpumpe als Blutpumpe verwendet, welche den Vorteil hat, dass die Flussrate bzw. der Mittelfluss bei einem höheren Druck, zumindest in der Diastole, niedrig sein kann und sowohl der Druck als auch die Flussrate in der diastolischen Phase erhöht werden können. Somit ist der verbleibende Druck nach dem Puls und vor der systolischen Phase möglichst gering, bevorzugt durch eine entsprechende Steuerung sogar negativ. Weiterhin hat eine Diagonalpumpe im Vergleich zu einer Zentrifugalpumpe den Vorteil, dass der Impeller einen geringeren Umfang aufweist und eine geringere Masse zu beschleunigen hat und somit schneller beschleunigt und entschleunigt und der Puls feiner eingestellt werden kann, weil eine geringere Massenträgheit vorliegt.

Zusätzlich zu dem empfangenen EKG-Signal kann die Steuer- und Regeleinheit weiter dazu eingerichtet sein, eine Messung eines Aortendrucks des unterstützten Patienten über einen vorgegebenen Zeitraum zu empfangen und den Pulsparameter bei einem vorgegebenen Aortendruck und Änderung des Aortendrucks einzustellen.

Somit kann der Aortendruck als Verifikation der vorgegebenen Pulsverzögerung und/oder der Pulsdauer dienen, wobei die Latenzzeit für die fluidische Transportzeit berücksichtigt werden kann. Auf diese Weise kann ein Benutzer der extrakorporalen Kreislaufunterstützung den genauen Zeitpunkt des Triggersignals manuell einstellen und an den gemessenen Aortendruck oder Auswurfleistung anpassen. Der gemessene Aortendruck oder Auswurfleistung kann jedoch optional auch eine Rückkopplung für die Steuer- und Regeleinheit darstellen, wobei der gemessene Aortendruck oder Auswurfleistung ein Feedbacksignal bereitstellt, welches beim Einstellen des Pulsparameters automatisch berücksichtigt wird. Somit kann das Einstellen des Pulsparameters weiter verbessert und iterativ erfolgen.

Alternativ kann der gemessene Aortendruck auch das EKG-Signal ersetzen, wobei der Aortendruck durch die Steuer- und Regeleinheit evaluiert wird und eine Änderung des Aortendrucks in einer Druckkurve charakteristisch für eine spezifische Herzphase des Herzzyklus ist. Beispielsweise kann ein kurzer Anstieg des Aortendrucks nach einem Abfall des Aortendrucks (Dikrot'scher Punkt) das Schließen der Aortenklappen und somit den Diastolenbeginn bzw. die Füllungsphase des Herzens indizieren. Eine solche Druckänderung kann beispielsweise in einer Druckkurve als Dikrot'scher Punkt detektiert werden.

Bevorzugt ist die Steuer- und Regeleinheit dazu eingerichtet, aus dem EKG-Signal und/oder des Aortendrucks eine diastolische Phase und systolische Phase des Herzens des unterstützten Patienten zu bestimmen und zumindest die Pulsdauer derart einzustellen, dass diese vor der systolischen Phase endet.

Während die R-Zacke des EKG-Signals den Systolenbeginn bzw. den Herzauswurf angibt, indiziert ein kurzer Anstieg des Aortendrucks nach einem Abfall des Aortendrucks das Schließen der Aortenklappen den Diastolenbeginn bzw. das Füllen des Herzens, wie vorstehend beschrieben. Entsprechend kann die Genauigkeit der Ausgabe des Signals zum Regeln der Blutpumpe durch eine Kombination des EKG-Signals und des gemessenen Aortendrucks weiter erhöht werden, sodass eventuelle EKG-Störungen die Sicherheit des Patienten und die Funktionalität der Steuer- und Regeleinheit nicht beeinträchtigen.

Das Beenden des Pulses vor der systolischen Phase ist, wie vorstehend beschrieben, vorteilhaft für die Nachlastsenkung und ermöglicht dennoch eine diastolische Augmentation bei einer hinreichenden Perfusion der Endorgane. Eine solche diastolische Augmentation kann beispielsweise durch einen retrograden Blutfluss bei einer entsprechenden Kanülenpositionierung erzielt werden, beispielsweise bei einer Positionierung im Aortenbereich oder in der *a. femoralis.* Abhängig vom Patienten kann jedoch auch ein zentrales Kanülieren erforderlich sein, sodass ein antegrader Blutfluss bereitgestellt wird. In diesem Fall können die Pulsparameter alternativ auch mit der systolischen Phase eingestellt werden, sodass mit der systolischen Phase gepulst wird.

Die oben gestellte Aufgabe wird weiterhin durch ein System zur extrakorporalen Kreislaufunterstützung eines Patienten gelöst. Entsprechend umfasst das System einen venösen Patientenzugang und einen arteriellen Patientenzugang, eine nicht-okklusive Blutpumpe, welche fluidisch mit dem venösen Patientenzugang und dem arteriellen Patientenzugang verbunden ist und zum Bereitstellen eines Blutflusses vom venösen Patientenzugang zum arteriellen Patientenzugang ausgelegt ist, und eine Schnittstelle zum Empfangen einer Messung eines arteriellen Drucks und eines EKG-Signals des Patienten. Erfindungsgemäß umfasst das System weiterhin eine vorstehend beschriebene Steuer- und Regeleinheit.

Um die nicht-okklusive Blutpumpe fluidisch mit dem venösen Patientenzugang und dem arteriellen Patientenzugang zu verbinden, können im System eine Ansaugleitung bzw. eine Förderleitung vorgesehen sein, welche jeweils ein inneres Lumen mit einem Eingangs- und Ausgangsöffnung aufweisen wodurch Blut vom venösen Patientenzugang zum arteriellen Patientenzugang über die Blutpumpe fließen kann, wenn die Blutpumpe im Betrieb ist bzw. betätigt wird. Die Ansaugleitung kann weiterhin über eine spezifische Abzweigung mit einem Fluidbehälter verbunden sein, beispielsweise einem Blutreservoir, sodass dem Patienten während des Betriebs des Systems weitere Fluide verabreicht werden können, beispielsweise Blut für eine Bluttransfusion oder zum Behalten eines hinreichenden Blutvolumens. Die Leitungen können mit der Blutpumpe über einfache Verbindungen wie Luer-Verbindungen verbunden werden und sind bevorzugt, wie auch die Blutpumpe, als austauschbare Einmalartikel ausgebildet. Die Blutpumpe kann dabei mit einem Pumpenantrieb über einen Pumpenkopf gekoppelt sein, beispielsweise mittels einer magnetischen Kopplung. Dadurch kann das System sukzessive für mehrere Patienten verwendet während gleichzeitig die Hygienevorschriften eingehalten werden.

Die Ansaugleitung und Förderleitung können weiterhin jeweils eine Kanüle aufweisen, sodass das System eine venöse Kanüle und eine arterielle Kanüle umfasst, welche dazu konfiguriert sind, in das Herz oder in die Herzregion des Patienten, beispielsweise in den Bereich des rechten Vorhofs bzw. den Aortenbereich, eingeführt zu werden. Dadurch können die Blutentnahme und die Blutabgabe genau auf die Anatomie des Herzens abgestimmt und ein pulsatiler Blutfluss genau im Bereich der Koronararterien bereitgestellt werden. Hierdurch wird der Auswurf in der systolischen Phase nicht beeinträchtigt während die Perfusion der Koronararterien in der diastolischen Phase optimiert wird.

Die Blutpumpe ist weiterhin bevorzugt eine Diagonalpumpe, wie vorstehend beschrieben. Dadurch kann eine geringe Flussrate bei einem höheren Druck bzw. mit höheren Druckspitzen bereitgestellt werden, was für die genaue Synchronisierung mit dem EKG-Signal, insbesondere zur Synchronisierung mit der diastolischen Phase, vorteilhaft ist. Weiterhin hat der Impeller einer solchen Diagonalpumpe einen geringeren Umfang und eine geringere Masse zu beschleunigen, sodass dieser somit schneller beschleunigt und entschleunigt und der Puls feiner eingestellt werden kann. Die Pumpenleistung bewirkt, dass ein Blutfluss von einer Seite mit einem niedrigen Druck zu einer Seite mit einem höheren Druck bereitgestellt wird, wobei die Steuer- und Regeleinheit dabei den Motor bzw. den Pumpenantrieb der Blutpumpe betätigt und dazu eingerichtet ist, die Drehzahl des Rotorblatts derart einzustellen, dass die Blutpumpe entsprechend einen wellenartigen Fluss erzeugt, welcher einem pulsatilen Blutfluss entspricht.

Das System kann durch das Bereitstellen eines synchronisierten pulsatilen Blutflusses insbesondere für die Behandlung einer Sauerstoffunterversorgung des Herzmuskelgewebes, beispielsweise von einem kardiogenen Schock, eingesetzt werden, sodass die Perfusion der Koronararterien auch in einem pathophysiologischen Zustand und bei einer reduzierten Sauerstoffversorgung des Herzmuskels verbessert wird, ohne die Perfusion der Endorgane zu beeinträchtigen bzw. diese gleichzeitig ebenfalls zu verbessern. Das System bietet somit bevorzugt eine therapeutische Unterstützung der patienteneigenen Herzleistung. Weiterhin kann das System bei Katheterisierungsvorgängen unterstützend oder auch im Falle eines Herzinfarkts oder Herzstillstands bzw.

bei unterschiedlicher oder ausbleibender Herzleistung als künstlicher Ersatz und Unterstützung der Herzfunktion eingesetzt werden.

Eine oder mehrere Komponenten des Systems können weiterhin in einem Gehäuse integriert sein. So kann die Steuer- und Regeleinheit beispielsweise in einer Konsole untergebracht sein, welche eine Benutzerschnittstelle zum Eingeben und Auslesen von Einstellungen des Systems, insbesondere von Parametern der Blutpumpe, aufweist. Beispielsweise kann die Konsole ein Touchscreen und/oder ein Display mit einer Tastatur umfassen, welche von einem Benutzer bedient werden können. Die Steuer- und Regeleinheit betreibt, betätigt, steuert, regelt und überwacht dabei die Blutpumpe und ermöglicht eine Synchronisierung der Blutpumpe mit dem Herzzyklus des jeweiligen Patienten.

Beispielsweise kann die Steuer- und Regeleinheit das empfangene EKG-Signal und die Herzfrequenz aufzeichnen, wobei das Display das aktuelle EKG-Signal grafisch und die aktuelle oder gemittelte Herzfrequenz numerisch wiedergibt. Weiterhin können charakteristische Eigenschaften des EKG-Signals in der grafischen Darstellung betont bzw. markiert werden, sodass in einem QRS-Signal beispielsweise eine zum Ausgeben des Pulsparameters bzw. des Pumpensignals erfasste R-Zacke im EKG-Signal markiert werden kann. Weiterhin können weitere Einstellungen wie der Zeitpunkt des ausgegebenen Pulsparameters und die Pulsdauer im EKG-Signal abgebildet sein, sodass ein Benutzer die Steuerung und Regelung der Blutpumpe im Hinblick auf den physiologischen Zustand des Patienten überwachen kann.

Die Schnittstelle kann beispielsweise als Sensorbox ausgebildet sein, welche über Anschlüsse mit verschiedenen Sensoren wie beispielsweise im Schlauchsystem integrierten Drucksensoren und einem EKG-Gerät verbunden werden kann.

Bevorzugt umfasst das System weiterhin ein EKG-Gerät, welches mit der Schnittstelle kommunikativ verbunden ist. Dadurch kann das System unabhängig vom Vorhandensein von anderen Komponenten eingesetzt werden. Das EKG-Gerät kann weiterhin an der Steuer- und Regeleinheit befestigt sein, sodass ein kompaktes System ermöglicht wird. Bevorzugt ist das EKG-Gerät dabei in einem einzelnen Gehäuse des Systems integriert, beispielsweise in der Sensorbox in Form einer EKG-Karte oder eines EKG-Moduls. Alternativ kann die Steuer- und Regeleinheit jedoch auch dazu eingerichtet sein, ein externes EKG-Signal des unterstützten Patienten zu empfangen, beispielsweise von einem außerhalb des Systems angeordneten Herzmonitors. Dadurch kann das System noch kompakter ausgebildet werden.

Weiterhin kann das System zur extrakorporalen Kreislaufunterstützung als tragbares System konfiguriert sein, sodass eine Behandlung eines Patienten nicht an einem festen Ort erfolgen muss und die Mobilität des Patienten verbessert wird. Dazu können die verschiedenen Komponenten des Systems wie die Blutpumpe, die Steuer- und Regeleinheit und ggf. das EKG-Gerät durch eine integrierte Batterie betrieben werden. Bevorzugt können jedenfalls die nicht-okklusive Blutpumpe und/oder die Steuer- und Regeleinheit, weiter bevorzugt beide, zur extrakorporalen Anordnung ausgelegt sein. Auch das EKG-Gerät kann bevorzugt zur extrakorporalen Anwendung ausgelegt sein.

Um ein genaueres Entnehmen des Blutes und eine genauere Abgabe des pulsatilen Blutflusses zu ermöglichen, umfasst das System bevorzugt mindestens eine Kanüle zum Einführen des arteriellen Patientenzugangs in den Patienten und eine Kanüle zum Einführen des venösen Patientenzugangs in den Patienten, wie vorstehend beschrieben. Durch die Kanülen kann ebenfalls das erforderliche EKG-Signal abgeleitet werden, beispielsweise über eine Ableitung an der Kanülenspitze oder an einer Stelle der venösen Kanüle, einer venösen Drainagekanüle im rechten Vorhof oder der *vena cava superior,* an der Kanülenspitze oder an einer Stelle der arteriellen Kanüle in der Aorta oder der *a. femoralis* oder der *a. iliaca.* Somit kann beispielsweise eine R-Zacke eines QRS-Signals genau erfasst werden und es werden Störungen, die bei einem Oberflächen-EKG auftreten können, weitestgehend vermieden.

Bevorzugt ist die Kanüle zum Einführen des arteriellen Patientenzugangs zum Einführen in den Aortenbereich, beispielsweise in die Aorta ascendens oder descendens, oder in die *a. femoralis,* und/oder ist die Kanüle zum Einführen des venösen Patientenzugangs zum Einführen in den rechten Vorhof oder in die *vena cava* ausgebildet. Es können somit - je nach physiologischem Zustand des Patienten - verschiedene Kanülengrößen und Bereiche des Patientenkreislaufs gewählt werden.

Alternativ können die Kanülen auch als gemeinsame Kanüle bzw. als Doppellumenkanüle ausgebildet sein, welche beispielsweise interkostal in den Apex des Herzens eingeführt werden kann. Eine solche Kanüle kann beispielsweise vorgesehen sein, wenn eine Einführung in den Aortenbereich erschwert oder unvorteilhaft für den Patienten ist. Es können somit - in Abhängigkeit patientenspezifischer Umstände - intravasale oder auch intrakardiale Kanülen im System vorgesehen sein.

Das System kann weiterhin einen Oxygenator oder Membranoxygenator aufweisen, welcher eine Dekarbolisierung, also Kohlendioxid-Entfernung oder -Abreicherung, und Oxygenierung des aus dem Patienten entnommenen Bluts ermöglicht und eine im Vergleich zu bekannten IABP Methoden erheblich bessere Sauerstoffversorgung des Herzmuskelgewebes bewirkt. Beispielsweise kann im System ein Membranventilator wie ein iLA-Membranventilator vorgesehen sein, welcher auch ohne Pumpe und nur durch die Herzleistung betrieben werden kann, da dieser einen geringen Widerstand aufweist. Bevorzugt umfasst das System einen im Vergleich zum Gehäuse relativ flachen und eckigen Oxygenator, welche gelegte Matten aufweist. Alternativ kann jedoch jeder Gasaustauscher vorgesehen sein, also auch ein runder und/oder zylindrischer Oxygenator, beispielsweise mit gewickelten Matten. Bevorzugt ist der Membranoxygenator dazu eingerichtet, extrakorporal angeordnet zu sein.

Die oben gestellte Aufgabe wird weiterhin durch ein Verfahren zum Steuern/Regeln einer nicht-okklusiven Blutpumpe einer extrakorporalen Kreislaufunterstützung gelöst. Das Verfahren umfasst zumindest die folgenden Schritte:

- Empfangen eines Flusswerts der extrakorporalen Kreislaufunterstützung;

- Empfangen einer Messung eines arteriellen Drucks und eines EKG-Signals eines unterstützten Patienten über einen vorgegebenen Zeitraum;

- Bestimmen eines mittleren arteriellen Drucks der extrakorporalen Kreislaufunterstützung oder des unterstützten Patienten anhand der Messung des arteriellen Drucks und eines energieäquivalenten Drucks anhand des Flusswerts und des arteriellen Drucks;

- Bestimmen eines erforderlichen Werts von mindestens einem Pulsparameter der Blutpumpe anhand des mittleren arteriellen Drucks, des energieäquivalenten Drucks und des EKG-Signals, um ein Verhältnis des energieäquivalenten Drucks zum mittleren arteriellen Druck zu bewirken, welches größer als 1,0 ist; und

- Einstellen des Pulsparameters in Abhängigkeit vom EKG-Signal.

Bevorzugt wird dabei aus dem EKG-Signal eine R-Zacke bestimmt und der Pulsparameter an einem vorgegebenen Zeitpunkt nach der R-Zacke eingestellt, wie vorstehend beschrieben. Dadurch wird eine Verbesserung der Perfusion der Koronararterien in der diastolischen Phase ermöglicht und die Nachlast signifikant reduziert.

Als Pulsparameter können dabei ebenfalls eine Pulsamplitude, eine Pumpendrehzahl, eine Pumpendrehzahländerung pro Zeiteinheit, eine Pulsdauer, eine systolische Pumpendauer, eine diastolische Pumpendauer, eine Antriebsradentschleunigung, eine Antriebsradbeschleunigung und/oder einen gemittelten Pumpenfluss eingestellt werden.

Weiterhin kann aus dem EKG-Signal eine diastolische Phase und systolische Phase des Herzens des unterstützten Patienten bestimmt und zumindest die Pulsdauer derart eingestellt werden, dass diese vor der systolischen Phase endet. Somit wird der Puls und der bereitgestellte energieäquivalente Druck an die Herzfrequenz des Patienten angepasst und die mögliche Perfusion der Koronararterien entsprechend optimiert.

Weiterhin kann vorgesehen sein, dass ein Wert über die Auswurfleistung empfangen wird, der entweder errechnet oder z.B. durch eine Impedanzmessung oder der Druckmessung ermittelt wird.

Wie oben beschrieben, kann - abhängig vom Patienten - auch ein zentrales Kanülieren erforderlich sein, sodass ein antegrader Blutfluss bereitgestellt wird. In diesem Fall können die Pulsparameter alternativ auch mit der systolischen Phase eingestellt werden, sodass mit der systolischen Phase gepulst wird.

Der erforderliche Wert des Pulsparameters kann weiterhin in Abhängigkeit von einem vorgegebenen Zielwert oder einem vorgegebenen Zielprozentwert des energieäquivalenten Drucks bestimmt werden.

So kann die Steuerung des Fluss-, Druck- und/oder Pulscharakteristik-Parameters auch über die Vorgabe eines Wertes des energieäquivalenten Drucks erfolgen. Beispielsweise kann der aktuelle energieäquivalenten Druckwert auf einem Display einer Konsole angezeigt sein und dann optional auf einen Zielwert verändert werden. Die Anzeige kann auch als Prozentwert des energieäquivalenten Drucks erfolgen, der sich auf den maximal möglichen Wert des energieäquivalenten Drucks bei dem individuellen Patienten bezieht. Dieser Maximalwert kann beispielsweise patientenindividuell durch einen kurzen empirischen Algorithmus ermittelt werden.

Das erfindungsgemäße Verfahren sieht bevorzugt vor, dass es mit einer erfindungsgemäßen Steuer- und Regeleinheit durchgeführt wird.

Weiter bevorzugt ist bei einem erfindungsgemäßen Verfahren die gesteuerte/geregelte nicht-okklusive Blutpumpe (40) fluidisch mit einem, typischerweise extrakorporal angeordneten Membranoxygenator verbunden. Hierdurch kann dem Patienten Sauerstoff-angereichertes und/ oder Kohlendioxid-abgereichertes (Eigen)blut nach Passage des Membranoxygenators zugeführt werden. Die Komponenten, durch die die Verfahrensschritte durchgeführt werden, sind typischerweise extrakorporal angeordnet. Hierdurch können einzelne, mehrere oder bevorzugt alle Verfahrensschritte des erfindungsgemäßen Verfahrens, wie in Anspruch 17 definiert, nicht-invasiv, sondern extrakorporal ausgeführt werden.

Weiterhin kann auf einem Display oder Monitor ebenfalls das Verhältnis des energieäquivalenten Drucks zum mittleren arteriellen Druck angezeigt sein. Bevorzugt wird der energieäquivalente Druck in mmHg und/oder als Prozentwert des maximal erreichbaren Werts des energieäquivalenten Drucks angezeigt.

## Kurze Beschreibung der Figuren

Bevorzugte weitere Ausführungsformen der Erfindung werden durch die nachfolgende Beschreibung der Figuren näher erläutert. Dabei zeigen:

Figur 1 ist eine schematische Darstellung einer Kontrolllogik einer Steuer- und Regeleinheit gemäß der Erfindung;

Figur 2 ist eine schematische Darstellung des arteriellen Druckverlaufs und des entsprechenden Flussverlaufs über einen vorgegebenen Zeitraum;

Figur 3 ist eine schematische Darstellung einer Pulsausgabe gemäß der Erfindung;

Figur 4 ist eine schematische Darstellung eines erfindungsgemäßen Systems zur extrakorporalen Kreislaufunterstützung;

Figur 5 ist eine schematische Darstellung einer erfindungsgemäßen Pulseinstellung anhand eines empfangenen EKG-Signals bei einer normofrequenten Herzfrequenz;

Figur 6 ist eine schematische Darstellung einer Überschneidung eines Patientenpulses und des Unterstützungspulses bei einer erhöhten Herzfrequenz;

Figuren 7A und 7B zeigen Beispiele von entsprechenden erfindungsgemäßen Pulsparametereinstellungen gemäß Figur 5 bzw. Figur 6; und

Figur 8 ist eine schematische Darstellung einer erfindungsgemäßen Pulseinstellung zur verbesserten Nachlastsenkung.

## Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente in den unterschiedlichen Figuren mit identischen Bezugszeichen versehen, und auf eine wiederholte Beschreibung dieser Elemente wird teilweise verzichtet, um Redundanzen zu vermeiden.

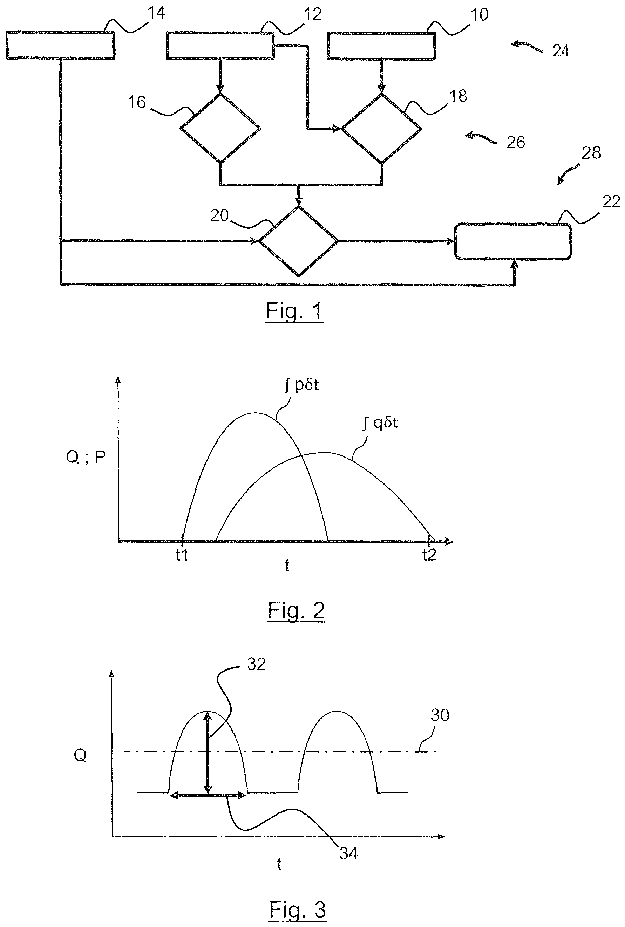

In Figur 1 ist schematisch eine Kontrolllogik für eine Steuer- und Regeleinheit gezeigt, wobei entsprechend Werte von einer Schnittstelle 24 in eine Steuer- und Regeleinheitsebene 26 eingegeben werden und nach Verarbeitung durch die Kontrolllogik eine Einstellung auf einer Blutpumpenebene 28 bewirken.

Entsprechend ist die Steuer- und Regeleinheit dazu eingerichtet über die Schnittstelle 24 einen Flusswert 10 der extrakorporalen Kreislaufunterstützung sowie eine Messung eines arteriellen Drucks 12 und eines EKG-Signals 14 eines unterstützten Patienten über einen vorgegebenen Zeitraum zu empfangen. Anhand der Messung des arteriellen Drucks 12 wird ein mittlerer arterieller Druck 16 bestimmt. Der mittlere arterielle Druck kann sowohl aus einer arteriellen Druckmessung 12 aus der extrakorporalen Kreislaufunterstützung als auch aus dem Patienten bestimmt werden, beispielsweise über einen Schlauchschenkel eines arteriellen Zugangs bzw. über einen invasiven Drucksensor. Gemäß der vorliegenden Ausführungsform wird der mittlere arterielle Druck 16 des unterstützten Patienten bestimmt, beispielsweise entweder über die *a. radialis* oder direkt in der Aorta.

Weiterhin kann ein Flusswert 10 über einen entsprechenden Schlauchschenkel des arteriellen Zugangs erfolgen und eine direkte Rückkopplung bezüglich des Blutflusses in der extrakorporalen Kreislaufunterstützung für die Steuer- und Regeleinheit bereitstellen. Beispielsweise kann der Blutfluss, der von einer nichtokklusiven Blutpumpe bereitgestellt wird, über eine Drehzahl eingegeben und an den Rückkopplungswert angepasst werden. Mit anderen Worten kann zunächst ein Flusswert 10 über eine entsprechende Drehzahl eingestellt und anhand einer aktuellen Messung kontinuierlich oder periodisch korrigiert werden. Dadurch können eventuelle unterschiedliche Patientenparameter oder auch Schlauchparameter, welche eine Änderung des Flusswerts 10 bei derselben Drehzahl der Blutpumpe bewirken können, berücksichtigt werden.

Die Steuer- und Regeleinheit bestimmt weiterhin einen energieäquivalenten Druck 18 anhand des empfangenen Flusswerts 10 und des arteriellen Drucks 16. Um einen Pulsbeitrag zum mittleren arteriellen Druck zu bewirken, wird in der Steuer- und Regeleinheit weiterhin ein aktuelles Verhältnis des energieäquivalenten Drucks 18 zum mittleren arteriellen Druck 16 ermittelt. Beispielsweise kann dieses Verhältnis zunächst etwa 1,0 sein, sodass kein Pulsbeitrag oder nur ein begrenzter Pulsbeitrag von der extrakorporalen Kreislaufunterstützung erzielt wird. Um das Verhältnis und somit den Pulsbeitrag zu erhöhen, wird in der Steuer- und Regeleinheit ein erforderlicher Wert von mindestens einem Pulsparameter 20 der Blutpumpe bestimmt. Dazu wird nicht nur iterativ das Verhältnis zwischen dem energieäquivalenten Druck 18 und dem mittleren arteriellen Druck 16 bestimmt, sondern es wird ebenfalls das empfangene EKG-Signal 14 berücksichtigt.

Beispielsweise kann aus dem EKG-Signal eine mögliche Pulsdauer ermittelt werden, welche eine Überlappung zweier Herzphasen des Herzzyklus während des Einstellens 22 des Pulsparameters 20 verhindert. Dadurch kann der Puls über die ermittelte mögliche Pulsdauer optimiert werden, um den erwünschten bzw. den erforderlichen Pulsbeitrag zu erzielen und somit das Verhältnis zwischen dem energieäquivalenten Druck 18 und dem mittleren arteriellen Druck 16 zu erhöhen, beispielsweise auf 1,1 oder höher. Als weiterer Pulsparameter kann beispielsweise eine Drehzahländerung pro Zeiteinheit gewählt werden, welche die Pulsamplitude während der ermittelten und somit vorgegebenen Pulsdauer derart erhöht, dass ein Puls mit einem vorgegebenen Volumen oder Fluss pro Zeiteinheit ausgegeben wird, der einen entsprechenden Beitrag zum mittleren arteriellen Druck bewirkt.

Die Pulsparameter 20 werden entsprechend auf den ermittelten Wert eingestellt 22 und zwar weiterhin anhand des EKG-Signals 14, sodass eine exakte zeitliche Vorgabe des ausgegebenen Pulses erfolgt. Dadurch kann beispielsweise eine diastolische Augmentation erfolgen, ohne dass die Nachlast erhöht bzw. diese so wenig wie möglich erhöht wird, zumal der Puls derart ausgegeben wird, dass dieser vor der systolischen Phase des Herzzyklus beendet und der verbleibende Druck somit sehr gering ist.

In Figur 2 ist ein Beispiel eines arteriellen Druckverlaufs (P) und des entsprechenden Flussverlaufs (Q) über einen vorgegebenen Zeitraum (t) gezeigt. Dabei können für ein Zeitintervall t1 bis t2 (t2 >t1, d.h. t1 liegt gegenüber t2 in der Vergangenheit) sowohl ein Integral der Druckänderung, beispielsweise in mmHg, als auch ein Integral der Änderung des Flusses, beispielsweise in l/min, berechnet werden, welche als Grundlage für die Berechnung des energieäquivalenten Drucks dienen, beispielsweise nach der folgenden Formel:

$$\text{EEP (mm Hg)} = \int_{t1}^{t2} fp\,dt \Big/ \int_{t1}^{t2} f\,dt$$

wobei der Blutfluss (f) und der Druck (p) über ein Zeitintervall von t1 bis t2 berücksichtigt werden.

Aus Figur 2 ist weiter ersichtlich, dass der Anstieg des Drucks und des Flusses zeitlich verschoben sein können. Mit anderen Worten kann die Flussänderung verzögert nach der Druckänderung erfolgen und somit eine Latenzzeit in der extrakorporalen Kreislaufunterstützung verursachen.

Um einen pulsatilen Blutfluss bereitzustellen, ist vorgesehen, dass mindestens ein Pulsparameter in Abhängigkeit von einem EKG-Signal eingestellt wird. Dadurch kann regelmäßig gepulst und der jeweilige Puls kann an den physiologischen Zustand des Patienten angepasst werden. Ein Beispiel solcher Pulsparameter ist in Figur 3 schematisch dargestellt.

Entsprechend sind zwei Pulse in regelmäßigem Abstand über Zeit (t) gezeigt, wobei die Pulse auf einen optionalen Grundfluss aufgerechnet werden. Der Grundfluss kann durch Einstellen der Drehzahl bewirkt werden, sodass ein Blutfluss zwischen beispielsweise etwa 2 l/min und 3 l/min bereitgestellt wird.

Der jeweilige Puls wird im vorliegenden Beispiel durch eine Drehzahländerung erreicht, welche durch Betätigen des Motors und des Pumpenantriebs in der Blutpumpe bewirkt wird. Dadurch entsteht eine Pulsamplitude 32, welche eine entsprechende Änderung des Druckes (P) und somit des Flusses (Q) bewirkt. Der Puls wird für eine vorgegebene Pulsdauer 34 ausgegeben, welche mit der ermittelten möglichen Pulsdauer übereinstimmt, sodass sich aus der Pulsdauer 34 und der Drehzahländerung pro Zeiteinheit ein maximaler Blutfluss bzw. eine maximale Pulsamplitude 32 ergibt. Nach Erreichen der Pulsamplitude 32 wird der Pumpenantrieb nicht mehr betätigt, sodass die Drehzahl der Blutpumpe nach der vorgegebenen bzw. eingestellten Pulsdauer 34 auf den Grundfluss reduziert wird. Aus den Pulsen und dem Grundfluss ergibt sich somit ein Mittelfluss 30, welcher mit der gestrichelten Linie dargestellt ist. Der Mittelfluss 30 ist somit größer als der Grundfluss und bewirkt einen zusätzlichen Beitrag zum Herzzeitvolumen, der den mittleren arteriellen Druck des Patienten erhöht und eine hinreichende Perfusion bzw. Oxygenierung der Endorgane ermöglicht.

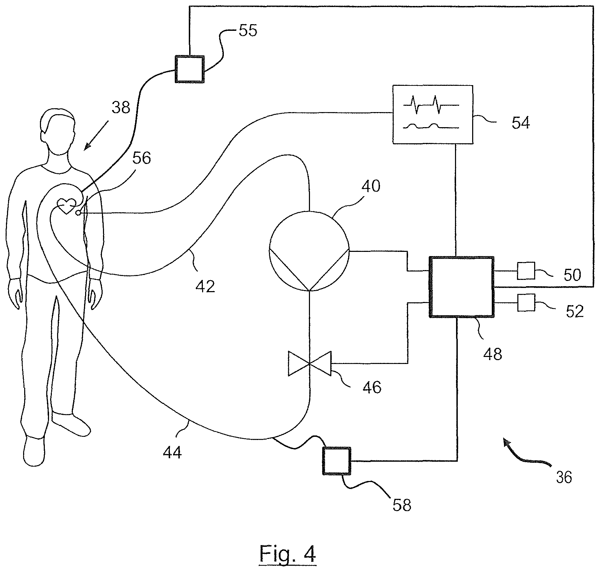

Ein System zur extrakorporalen Kreislaufunterstützung 36 ist schematisch in Figur 4 gezeigt. Das System 36 ist mit dem Patienten 38 verbunden und ermöglicht ein extrakorporaler Blutfluss mittels einer nicht-okklusiven Blutpumpe 40. Entsprechend ist die Blutpumpe 40 mit dem Kreislauf des Patienten 38 über einen venösen Patientenzugang 42 und einen arteriellen Patientenzugang 44 verbunden, sodass Blut vom Patienten 38 über eine Ansaugleitung entnommen und dem Patienten 38 über eine Förderleitung mit höherem Druck zurückgeführt wird. Die Patientenzugänge 42, 44 sind dabei optional als Kanülen ausgebildet und werden derart eingeführt, dass eine venöse Blutentnahmestelle (Kanülenspitze und Kanülenschaft) im Bereich des rechten Vorhofs, bspw. in der *v. cava superior* und/oder *v. cava inferior,* angeordnet ist und die arterielle Rückgabe über einen femoralen Patientenzugang 44 erfolgt, der entweder in die *a. femoralis* oder in dem Aortenbereich angeordnet ist, um einen retrograden Blutfluss bereitzustellen. Weiterhin kann stromabwärts von der Blutpumpe 40 ein optionaler Flussbegrenzer 46 vorgesehen sein. Obwohl dies nicht in Figur 4 dargestellt ist, kann das System 36 weiterhin einen Oxygenator zum Oxygenieren und/oder zur $CO_2$-Reduzierung des venösen Blutes umfassen, welcher beispielsweise in Flussrichtung stromabwärts von der Blutpumpe 40 angeordnet sein kann.

Die Blutpumpe 40 wird von einer Steuer- und Regeleinheit 48 betrieben bzw. geregelt. Die Steuer- und Regeleinheit 48 kann als Konsole ausgebildet sein, welche eine Benutzerschnittstelle 50 und einen Monitor 52 umfasst, über welche Einstellungen vorgenommen bzw. Informationen ausgegeben werden können. Somit können Parameter des Systems 36 von einem Benutzer geändert und/oder überwacht werden.

Die Steuer- und Regeleinheit 48 ist weiterhin mit einem EKG-Gerät gekoppelt, sodass ein EKG-Signal 54 empfangen werden kann. Das EKG-Gerät kann beispielsweise in einer Schnittstelle, wie einer Sensorbox des Systems, als EKG-Karte oder EKG-Modul eingebaut sein, kann alternativ aber auch als externes Gerät mit dem System über eine Schnittstelle gekoppelt werden. Das EKG-Signal 54 wird durch an den Patienten 38 angebrachte Sensoren 56 bereitgestellt, beispielsweise als 4-poliges Oberflächen-EKG und kann in Einkanal- oder Mehrkanaldarstellung mit Ableitungswahl dargestellt werden, beispielsweise eine Goldbergerableitung und/oder Extremitätenableitung.

Obwohl die Sensoren 56, hier lediglich schematisch dargestellt, ebenfalls einen Drucksensor umfassen können, welcher an einem anderen anatomischen Bereich des Patienten angeordnet sein kann, ist in der vorliegenden Ausführungsform ein Drucksensor vorgesehen, der einen arteriellen Druck des Patienten 38 misst. Der Drucksensor kann beispielsweise an einer arteriellen Kanüle gekoppelt oder als eigenständige Kanüle beispielsweise in die *a. radialis* eingebracht werden. Der Drucksensor gibt somit eine Druckmessung 55 aus, welche von der Steuer- und Regeleinheit 48 empfangen wird.

Wie vorstehend beschrieben, kann die Druckmessung 55 jedoch ebenfalls beispielsweise rechnerisch bestimmt werden oder über eine Schlauchleitung bzw. einen Schlauchschenkel einer arteriellen Kanüle oder Förderleitung erfolgen, um einen entsprechenden Druckwert der extrakorporalen Kreislaufunterstützung zu erfassen. Optional können ebenfalls mehrere im Schlauchset eingebaute Drucksensoren vorgesehen sein, wobei bevorzugt ein Drucksensor stromaufwärts der Blutpumpe 40, ein Drucksensor stromabwärts der Blutpumpe 40 und ggf. ein weiterer Drucksensor stromabwärts von einem optional vorgesehenen Oxygenator angeordnet sind.

Die Steuer- und Regeleinheit 48 ist dazu eingerichtet, einen mittleren arteriellen Druck des Patienten 38 basierend auf der empfangenen Druckmessung 55 zu bestimmen, bevorzugt kontinuierlich und in Echtzeit. Der mittlere arterielle Druck kann dabei als Richtwert für den von der Blutpumpe 40 bereitgestellten Blutfluss dienen, um eine hinreichende Perfusion der Endorgane zu bewirken. Ein zu hoher Blutfluss kann jedoch dazu führen, dass der retrograde Blutfluss die Aortenklappe verschließt und somit die Nachlast für die systolische Phase erhöht bzw. den linken Ventrikel am Auswurf hindert, wenn dieser zum falschen Zeitpunkt des Herzzyklus abgegeben wird. Dadurch erhöht sich der Druck im linken Ventrikel, was durch eine Erhöhung der Wandspannung des linken Ventrikels eine Einschränkung der Koronardurchblutung mit sich bringt. Gleichzeitig ergibt sich ein Rückstau über die *v. pulmonalis* in Richtung Lunge, was zu einem Lungenödem und einer pulmonalen Minderfunktion führen kann.

Um den Blutfluss entsprechend weiter an den Patienten 38 anzupassen, ist die Steuer- und Regeleinheit 48 dazu eingerichtet, einen Flusswert der extrakorporalen Kreislaufunterstützung von einem Flusssensor 58 zu empfangen, wobei der Flusssensor 58 an einem Schlauchschenkel des arteriellen Patientenzugangs 44 angebracht ist. Der Flusssensor 58 stellt somit eine Rückkopplung für die eingestellte Drehzahl an der Blutpumpe 40 bereit. Weiterhin ermöglicht der Flusswert, dass die Steuer- und Regeleinheit 48 neben dem mittleren arteriellen Druck ebenfalls den energieäquivalenten Druck bestimmen kann, wie vorstehend beschrieben. Dadurch kann die Steuer- und Regeleinheit 48 das Verhältnis zwischen dem aktuellen energieäquivalenten Druck und dem aktuellen mittleren arteriellen Druck bestimmen und iterativ den für eine Erhöhung des Verhältnisses über 1,0 erforderlichen Wert eines Pulsparameters bestimmen.

Dabei wird ebenfalls eine aus dem EKG-Signal 54 bestimmte Herzfrequenz sowie eine mögliche daraus resultierende Pulsdauer berücksichtigt, sodass eine Überlappung des Pulses mit einer herzeigenen systolischen Phase verhindert werden kann. Die Steuer- und Regeleinheit 48 stellt den so ermittelten, mindestens einen Pulsparameter entsprechend ebenfalls in Abhängigkeit vom EKG-Signal 54 ein, beispielsweise anhand der aus dem EKG-Signal 54 bestimmten R-Zacken.

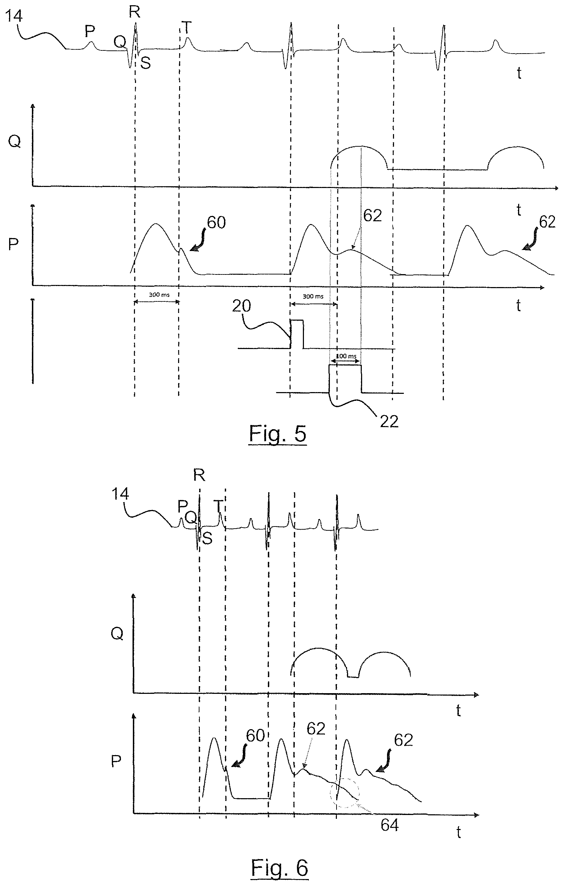

Eine solche Pulseinstellung ist schematisch in Figur 5 bei einer normofrequenten Herzfrequenz des unterstützten Patienten dargestellt. Vom Patienten wird kontinuierlich über Zeit (t) ein EKG-Signal 14 empfangen, welches einen QRS-Komplex umfasst. Obwohl grundsätzlich jede Amplitudenänderung im EKG Signal als Triggersignal für die Blutpumpe dienen kann, wird in der vorliegenden Ausführungsform die R-Zacke verwendet, welche den Herzauswurf bzw. die systolische Phase des Herzzyklus kennzeichnet. Dies wird auch in der dritten Zeile gezeigt, welche den arteriellen Druckverlauf im Patienten im Verlauf über Zeit (t) darstellt. Entsprechend findet unmittelbar nach der R-Zacke eine Änderung des arteriellen Drucks infolge des Herzauswurfs statt, welche durch die ersten beiden gestrichelten Linien gekennzeichnet ist und beispielsweise 300 ms betragen kann. Nach einer anfänglichen Druckerhöhung nimmt der Druck wieder ab, bis die Aortenklappen sich schließen, was eine geringe Druckerhöhung verursacht und durch den Dikrot'schen Punkt (Schluss der Aortenklappe) gekennzeichnet wird. Ab diesem Punkt beginnt die diastolische Phase bzw. die Füllungsphase des Herzens.

Wie in Figur 5 gezeigt, nimmt der Druck bei einem nicht-augmentierten Druck weiter ab, was durch den Pfeil mit dem Bezugszeichen 60 gekennzeichnet ist. Mit anderen Worten findet für diesen Herzschlag keine Unterstützung oder diastolische Augmentation statt bzw. wird hier nicht durch eine extrakorporale Kreislaufunterstützung gepulst, wie dies auch durch die Abwesenheit eines Pulses im Verlauf des Flusses (Q) der extrakorporalen Kreislaufunterstützung in der zweiten Zeile gezeigt wird. Die Füllung des Herzens wird somit nicht unterstützt, sodass der arterielle Druck im Patienten rasch abnimmt.

Nachdem die Herzfrequenz und auch die jeweilige Verzögerung zwischen der R-Zacke und dem Schließen der Aortenklappen bestimmt bzw. ermittelt worden sind, gibt die Steuer- und Regeleinheit ein Signal an den Pumpenantrieb bzw. den Motor der Blutpumpe aus, um einen Pulsparameter der Blutpumpe entsprechend einzustellen. Dabei wird die R-Zacke als Triggersignal und zum Bestimmen des Pulsparameters 20 verwendet und der jeweilige Pulsparameter 20 nach einer entsprechenden Verzögerung eingestellt 22, wie in der vierten Zeile schematisch dargestellt. Entsprechend werden Pulse in der extrakorporalen Kreislaufunterstützung bereitgestellt, welche derart zeitlich aufeinander abgestimmt sind, dass diese einen entsprechenden pulsatilen Blutfluss während der diastolischen Phase bewirken, wie in der zweiten Zeile gezeigt. Dadurch erfolgt eine Druckerhöhung während der Diastole, die in der Berechnung des mittleren arteriellen Drucks mit dem Faktor 2 gegen dem Faktor 1 des Druckes in der Diastole eingeht und somit einen 2-Fach höheren Beitrag zur Erhöhung des mittleren arteriellen Drucks liefert. Der somit erreichte augmentierte Druck 62 startet idealerweise nach dem Dikrot'schen Punkt und bewirkt somit die diastolische Augmentation, ohne jedoch mit der systolischen Phase zu überlappen.

Bei einer erhöhten Herzfrequenz kann sich der Unterstützungspuls jedoch mit der systolischen Phase des Patientenpulses überlagern oder die Abklingphase der Augmentation interferiert mit der Anstiegsphase des linkventrikulären Auswurfs, der Systole, wie dies in Figur 6 schematisch dargestellt ist. Bei einer erhöhten Herzfrequenz ist der Abstand zwischen den jeweiligen R-Zacken und somit auch zwischen dem Dikrot'schen Punkt und der nachfolgenden R-Zacke verkürzt, sodass die diastolische Phase ebenfalls verkürzt ist. Bei einer gleichbleibenden Pulsdauer kann der augmentierte Druck 62, der vom jeweiligen Puls bereitgestellt wird, mit der nachfolgenden systolischen Phase überlappen bzw. interferieren, was durch die Pulsüberlagerung 64 dargestellt wird. Dies führt zu einer Erhöhung der Nachlast und erfordert entweder eine Anpassung der Unterstützungsfrequenz oder eine Anpassung der jeweiligen Pulsparameter, unter Berücksichtigung der Beibehaltung eines ausreichenden, bzw. physiologischen EEP.

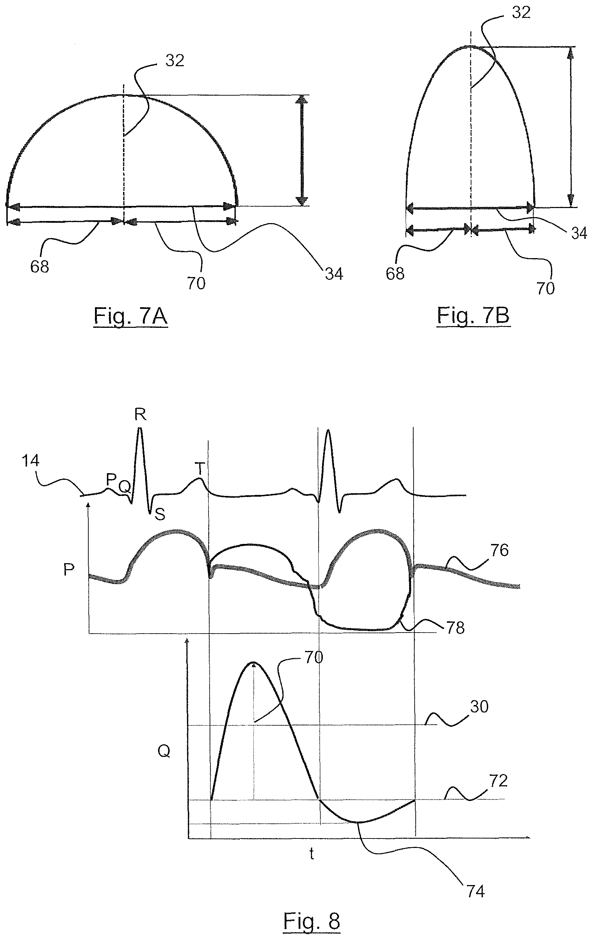

Obwohl eine Anpassung der Pulsfrequenz, etwa bei einer Tachykardie, erforderlich sein kann, werden bevorzugt die Pulsparameter bei einer erhöhten Herzfrequenz an den jeweiligen physiologischen Zustand des Patienten angepasst, wie dies in den Figuren 7A und 7B schematisch gezeigt ist.

So kann der Puls bei einer normofrequenten Herzfrequenz, wie in Figur 7A gezeigt, über eine längere diastolische Phase verteilt werden, sodass eine entsprechende längere Pulsdauer 34 von beispielsweise etwa 200 ms eingestellt werden kann. Gleichzeitig kann die Drehzahländerung pro Zeiteinheit in einem entsprechenden Bereich liegen, beispielsweise zwischen 2500 U/min und 4500 U/min gewählt werden, sodass die Pulsamplitude 32 ebenfalls in diesem entsprechenden Bereich liegt. Die Pulsdauer 34 kann weiterhin symmetrisch in eine gleichdauernde systolische Pulsphase 66 und diastolische Pulsphase 68 von jeweils etwa 100 ms eingeteilt sein, sodass ein gleichmäßiger Puls ausgegeben wird.

Bei einer erhöhten Herzfrequenz können die Pulsparameter jedoch modifiziert werden, wie in Figur 7B gezeigt. Die Pulsdauer 34 kann somit verkürzt werden und beispielsweise etwa 120 ms betragen. Um dennoch einen hinreichenden Beitrag zum mittleren arteriellen Druck zu bewirken, sofern der EEP im physiologischen Bereich liegt, kann dabei ebenfalls eine Drehzahländerung zwischen 3000 U/min und 5000 U/min gewählt werden, um die Pulsamplitude 32 und den Blutfluss während des Pulses entsprechend zu erhöhen.

Dadurch kann auch bei einer Herzfrequenz zwischen beispielsweise 90 und 130 Schläge pro Minute eine Flusskollision zwischen dem Pumpenauslauf und dem Systolenbeginn bzw. dem Herzauswurf verhindert und die Nachlast entsprechend optimiert werden. Weiterhin kann der Puls asymmetrisch eingeteilt sein, wobei die systolische Phase etwa 50% bis etwa 90% umfassen kann (nicht gezeigt).

Eine weitere Anpassung an höhere Herzfrequenzen zur Beibehaltung eines entsprechenden Mittelflusses kann durch die Herzfrequenzabhängige Variation des Unterstützungsverhältnisses auf 1:2 und 1:3 erreicht werden.

Die Anpassung der Pulsparameter an den physiologischen Zustand des Patienten hat somit den Vorteil, dass auch bei einer höheren Herzfrequenz die Nachlast nicht erhöht, ein adäquater mittlerer arterieller Druck und eine hinreichende Perfusion der Koronararterien ermöglicht werden kann. Die Pulsparameter sind weiterhin derart gewählt, dass ein Pulsbeitrag zum mittleren arteriellen Druck und ein EEP einer physiologischen Pulsqualität erreicht werden, sodass durch den pulsatilen Blutfluss eine verbesserte Perfusion und ein verbesserter Schutz der Endorgane bewirkt wird.

Eine weitere Verbesserung der Nachlastsenkung kann ebenfalls durch eine entsprechende Pulseinstellung erfolgen, wie schematisch in Figur 8 dargestellt. In dieser Darstellung sind das Triggersignal und die Steuer- und Regelsignale zur verbesserten Darstellung nicht gezeigt. Die Steuer- und Regeleinheit kann den Pulsparameter jedoch ähnlich wie in Figur 5 gezeigt einstellen, sodass auch in Figur 8 die R-Zacke aus dem QRS-Komplex des EKG-Signal 14 als Triggersignal dient und die Pulsabgabe zum Dikrot'schen Punkt erfolgt, so dass hierdurch eine diastolische Augmentation bewirkt wird.

Der arterielle Druck (P), beispielsweise gemessen an einem arteriellen Schenkel eines Schlauchsets, ist dabei beispielhaft und schematisch in der zweiten Zeile in Figur 8 sowohl ohne Augmentation 76 als auch mit Augmentation 78 gezeigt, wobei in der dritten Zeile schematisch eine Flusskurve bzw. die Änderung des Flusses (Q) in der extrakorporalen Kreislaufunterstützung während der Augmentation 78 gezeigt ist. Entsprechend erfolgt ohne Augmentation 76 eine rasche Senkung des arteriellen Drucks, ähnlich wie beim nicht-augmentierten Druck 60 in Figur 5. Es findet beim Ausgeben des Pulses eine Druckerhöhung und eine langsamere Drucksenkung bei Augmentation 78 statt, ähnlich wie beim augmentierten Druck 62 in Figur 5. Der Druckabfall kann jedoch auch anders aussehen, sodass die Form der Druckkurven lediglich als schematisches Beispiel zu verstehen ist und in jedem Patienten anders aussehen kann, beispielsweise in Abhängigkeit von patientenbezogenen Faktoren wie Gefäßelastizität, Verkalkungsgrad, Herzkraft, Viskosität, etc.

Der entsprechende Fluss (Q) in der extrakorporalen Kreislaufunterstützung wird auf einen Grundfluss 72 aufgerechnet und ergibt durch den Puls einen maximalen Flusswert, welcher durch die Drehzahländerung pro Zeiteinheit und die Pulsdauer vorgegeben ist und hierdurch eine diastolische Augmentation 70 gegenüber dem Grundfluss 72 bewirkt.

Zur Nachlastsenkung wird die Drehzahl nach der diastolischen Pumpenphase (systolische Herzphase) weiter unter den entsprechenden Wert des Grundflusses 72 eingestellt, sodass sich ein Mindestfluss 74 unter dem Grundfluss 72 ergibt und der Druck in der systolischen Phase des Herzens entsprechend reduziert wird, wie sich ebenfalls aus der Drucksenkung während der systolischen Phase bei der Augmentationskurve 78 ergibt. Dadurch wird weiterhin ein pulsatiler Blutfluss bei einem erforderlichen Mittelfluss 30 und einem Grundfluss 72 bereitgestellt, während gleichzeitig die Nachlast weiter reduziert werden kann. Dies kann, wie in Darstellung gezeigt, bezogen auf den nächsten Herzauswurf erflogen, aber auch auf den Übernächsten bei 1:2 Unterstützungsverhältnis.

Obwohl dies nicht in Figur 8 gezeigt ist, kann die Drehzahl alternativ auch derart geändert werden, dass ein negativer Blutfluss entsteht. Beispielsweise kann ein solcher negative Blutfluss durch eine Vierquadrantensteuerung vorgesehen sein, wie vorstehend beschrieben. Eine solche Vierquadrantensteuerung ermöglicht nämlich nicht nur eine Reduzierung (Bremsung) der Drehzahl bzw. des Impellers, sondern ermöglicht ebenfalls eine Umkehrung der Drehrichtung durch eine Umkehrung der Stromrichtung, so dass der Impeller in eine entgegengesetzte Richtung drehen kann. Ein solcher negative Blutfluss am Ende des Pulses und während der systolischen Phase bewirkt somit eine Ansaugwirkung, welche den Herzauswurf weiter erleichtert und somit die Nachlast für das beanspruchte und unterstützte Herz weiter reduziert.

Bezugszeichenliste

| | |
|---|---|
| 10 | Flusswert |
| 12 | arterieller Druckmessung |
| 14 | EKG-Signal |
| 16 | Bestimmen des mittleren arteriellen Drucks |
| 18 | Bestimmen des energieäquivalenten Drucks |
| 20 | Bestimmen des Pulsparameters der Blutpumpe |
| 22 | Einstellen des Pulsparameters |
| 24 | Schnittstelle |
| 26 | Steuer- und Regeleinheitsebene |
| 28 | Blutpumpenebene |
| 30 | Mittelfluss |
| 32 | Pulsamplitude |
| 34 | Pulsdauer |

36    System zur extrakorporalen Unterstützung
38    Patient
40    Blutpumpe
42    venöser Patientenzugang
44    arterieller Patientenzugang
46    optionaler Flussbegrenzer
48    Steuer- und Regeleinheit
50    Benutzerschnittstelle
52    Monitor
54    EKG-Signal
55    Druckmessung
56    Sensoren
58    Flusssensor
60    nichtaugmentierter Druck
62    augmentierter Druck
64    Pulsüberschneidung
66    systolische Pulsphase oder Pumpendauer
68    diastolische Pulsphase oder Pumpendauer
70    diastolische Augmentation
72    Grundfluss
74    Mindestfluss
76    Druck ohne Augmentation
78    Druck mit Augmentation

**Patentansprüche**

1. Steuer- und Regeleinheit (48) für eine nicht-okklusive Blutpumpe (40) einer extrakorporalen Kreislaufunterstützung, welche dazu eingerichtet ist,

   - einen Flusswert (10) der extrakorporalen Kreislaufunterstützung zu empfangen;
   - eine Messung eines arteriellen Drucks (12) und eines EKG-Signals (14) eines unterstützten Patienten (38) über einen vorgegebenen Zeitraum zu empfangen;
   - einen mittleren arteriellen Druck (16) der extrakorporalen Kreislaufunterstützung oder des unterstützten Patienten anhand der Messung des arteriellen Drucks (12) und einen energieäquivalenten Druck (18) anhand des Flusswerts (10) und des arteriellen Drucks (16) zu bestimmen; und
   - einen erforderlichen Wert von mindestens einem Pulsparameter (20) der Blutpumpe (40) anhand des mittleren arteriellen Drucks (16), des energieäquivalenten Drucks (18) und des EKG-Signals (14) zu bestimmen, um ein Verhältnis des energieäquivalenten Drucks (18) zum mittleren arteriellen Druck (16) zu bewirken, welches größer als 1,0 ist,

   wobei die Steuer- und Regeleinheit (48) weiterhin dazu eingerichtet ist, den Pulsparameter (20) in Abhängigkeit vom EKG-Signal (14) einzustellen (20).

2. Steuer- und Regeleinheit (48) gemäß Anspruch 1, wobei der mindestens eine Pulsparameter (20) ausgewählt ist aus einer Pulsamplitude (32), einer Pumpendrehzahl, einer Pumpendrehzahländerung pro Zeiteinheit, einer Pulsdauer (34), einer systolischen Pumpendauer (66), einer diastolischen Pumpendauer (68), einer Antriebsradentschleunigung, einer Antriebsradbeschleunigung und/oder einem gemittelten Pumpenfluss.

3. Steuer- und Regeleinheit (48) gemäß Anspruch 1 oder 2, welche weiter dazu eingerichtet ist, den erforderlichen Wert des Pulsparameters (20) anhand eines vorgegebenen mittleren arteriellen Drucks (16) und/oder eines vorgegebenen energieäquivalenten Drucks (18) zu bestimmen,
   und/oder
   welche weiter dazu eingerichtet ist, den Flusswert (10) anhand einer eingegebenen Drehzahl der Blutpumpe (40) oder automatisch anhand einer empfangenen Flussmessung zu bestimmen.

4. Steuer- und Regeleinheit (48) gemäß einem der vorstehenden Ansprüche, wobei der Flusswert (10) einen patientenspezifischen Mittelfluss (30) umfasst, wobei die Steuer- und Regeleinheit (48) weiter dazu eingerichtet ist, den Mittelfluss (30) anhand einer empfangenen Flussmessung und des mittleren arteriellen Drucks (16) durch Einstellen (22) des Pulsparameters (20) einzustellen (22),
   und/oder
   welche weiter dazu eingerichtet ist, aus dem EKG-Signal (14) eine Herzfrequenz zu bestimmen und den Pulsparameter (20) in Abhängigkeit von der Herzfrequenz einzustellen (22).

5. Steuer- und Regeleinheit (48) gemäß einem der vorstehenden Ansprüche, welche weiter dazu eingerichtet ist, aus dem EKG-Signal (14) eine Amplitudenänderung zu bestimmen und den Pulsparameter (20) an einem vorgegebenen Zeitpunkt nach der Amplitudenänderung einzustellen (22),
   welche bevorzugt dazu eingerichtet ist, aus dem EKG-Signal (14) eine R-Zacke zu bestimmen und den Pulsparameter (20) an einem vorgegebenen Zeitpunkt nach der R-Zacke einzustellen (22).

6. Steuer- und Regeleinheit (48) gemäß einem der vorstehenden Ansprüche, welche weiter dazu eingerichtet ist, eine Messung eines Aortendrucks des unterstützten Patienten (38) über einen vorgegebenen Zeitraum zu empfangen und den Pulsparameter (20) bei einem vorgegebenen Aortendruck und Änderung des Aortendrucks einzustellen (22).

7. Steuer- und Regeleinheit (48) gemäß einem der vorstehenden Ansprüche 5 bis 6, welche weiter dazu eingerichtet ist, aus dem EKG-Signal (14) und/oder dem Aortendruck eine diastolische Phase und systolische Phase des Herzens des unterstützten Patienten (38) zu bestimmen und zumindest die Pulsdauer (34) derart einzustellen (22), dass diese vor der systolischen Phase endet.

8. System (36) zur extrakorporalen Kreislaufunterstützung eines Patienten (38), umfassend:

    - einen venösen Patientenzugang (42) und einen arteriellen Patientenzugang (44),
    - eine nicht-okklusive Blutpumpe (40), welche fluidisch mit dem venösen Patientenzugang (42) und dem arteriellen Patientenzugang (44) verbunden ist und zum Bereitstellen eines Blutflusses vom venösen Patientenzugang (42) zum arteriellen Patientenzugang (44) ausgelegt ist,
    - eine Schnittstelle (24) zum Empfangen einer Messung eines arteriellen Drucks (12) und eines EKG-Signals (14; 54) des Patienten (38), und
    - eine Steuer- und Regeleinheit (48) nach einem der vorstehenden Ansprüche 1 bis 7.

9. System (36) gemäß Anspruch 8, ferner umfassend einen Membranoxygenator und/oder einen Membranventilator, bevorzugt einen extrakorporal angeordneten Membranoxygenator.

10. System (36) gemäß Anspruch 8 oder 9, ferner umfassend ein EKG-Gerät, welches mit der Schnittstelle (24) kommunikativ verbunden ist, und/oder wobei die nicht-okklusive Blutpumpe (40) und/oder die Steuer- und Regeleinheit (48), bevorzugt beide zur extrakorporalen Anordnung ausgelegt sind.

11. System (36) gemäß einem der vorstehenden Ansprüche 8 bis 10, ferner umfassend mindestens eine Kanüle zum Einführen des arteriellen Patientenzugangs (44) in den Patienten (38) und eine Kanüle zum Einführen des venösen Patientenzugangs (42) in den Patienten (38), wobei die Kanüle bevorzugt zum Einführen des arteriellen Patientenzugangs (44) zum Einführen in den Aortenbereich oder in die *a. femoralis* und/oder die Kanüle zum Einführen des venösen Patientenzugangs (42) zum Einführen in den rechten Vorhof oder in die *vena cava* ausgebildet ist.

12. Verfahren zum Steuern/Regeln einer nicht-okklusiven Blutpumpe einer extrakorporalen Kreislaufunterstützung, umfassend die Schritte:

    - Empfangen eines Flusswerts (10) der extrakorporalen Kreislaufunterstützung;
    - Empfangen einer Messung eines arteriellen Drucks (12) und eines EKG-Signals (14) eines unterstützten Patienten (38) über einen vorgegebenen Zeitraum;
    - Bestimmen eines mittleren arteriellen Drucks (16) der extrakorporalen Kreislaufunterstützung oder des unterstützten Patienten anhand der Messung des arteriellen Drucks (12) und eines energieäquivalenten Drucks (18) anhand des Flusswerts (10) und des arteriellen Drucks (12);
    - Bestimmen eines erforderlichen Werts von mindestens einem Pulsparameter (20) der Blutpumpe anhand des mittleren arteriellen Drucks (16), des energieäquivalenten Drucks (18) und des EKG-Signals (14), um ein Verhältnis des energieäquivalenten Drucks (18) zum mittleren arteriellen Druck (16) zu bewirken, welches größer als 1,0 ist; und
    - Einstellen (22) des Pulsparameters (20) in Abhängigkeit vom EKG-Signal (14).

13. Verfahren gemäß Anspruch 12, wobei aus dem EKG-Signal (14) eine R-Zacke bestimmt und der Pulsparameter (20) an einem vorgegebenen Zeitpunkt nach der R-Zacke eingestellt (22) wird.

14. Verfahren gemäß Anspruch 12 oder 13, wobei der mindestens eine Pulsparameter (20) ausgewählt ist aus einer Pulsamplitude (32), einer Pumpendrehzahl, einer Pumpendrehzahländerung pro Zeiteinheit, einer Pulsdauer (34), einer systolischen Pumpendauer (66), einer diastolischen Pumpendauer (68), einer Antriebsradentschleunigung, einer Antriebsradbeschleunigung und/oder einem gemittelten Pumpenfluss, und/oder

    wobei aus dem EKG-Signal (14) eine diastolische Phase und systolische Phase des Herzens des unterstützten Patienten (38) bestimmt und zumindest die Pulsdauer (32) derart eingestellt (22) wird, dass diese vor der systolischen Phase endet, und/oder wobei der erforderliche Wert des Pulsparameters (20) weiterhin in Abhängigkeit von einem vorgegebenen Zielwert oder einem vorgegebenen Zielprozentwert des energieäquivalenten Drucks (18) bestimmt wird.

15. Verfahren gemäß einem der vorstehenden Ansprüche 12 bis 14, wobei die nicht-okklusive Blutpumpe (40) fluidisch mit einem Membranoxygenator verbunden ist.

16. Verfahren gemäß einem der vorstehenden Ansprüche 12 bis 15, wobei die Verfahrensschritte, wie in Anspruch 12 definiert, nicht-invasiv durchgeführt werden.

17. Verfahren gemäß einem der vorstehenden Ansprüche 12 bis 16, wobei das Verfahren mit einer Steuer- und Regeleinheit (48) nach einem der Ansprüche 1 bis 7 durchgeführt wird.


**Claims**

1. Control and regulation unit (48) for a non-occlusive blood pump (40) of an extracorporeal circulatory support, configured for

   - receiving a flow value (10) of the extracorporeal circulatory support;
   - receiving a measurement of an arterial pressure (12) and an ECG signal (14) of a supported patient (38) over a predetermined period of time;
   - determining a mean arterial pressure (16) of the extracorporeal circulatory support or of the supported patient from the measurement of the arterial pressure (12) and an energy equivalent pressure (18) from the flow value (10) and the arterial pressure (16); and
   - determining a required value of at least one pulse parameter (20) of the blood pump (40) from the mean arterial pressure (16), the energy equivalent pressure (18), and the ECG signal (14) so as to effect a ratio of the energy equivalent pressure (18) to the mean arterial pressure (16) of greater than 1.0,

   wherein the control and regulation unit (48) is further configured to adjust (22) the pulse parameter (20) in dependence on the ECG signal (14).

2. Control and regulation unit (48) according to claim 1, wherein the at least one pulse parameter (20) is selected from a pulse amplitude (32), a pump speed, a pump speed change per unit time, a pulse duration (34), a systolic pump duration (66), a diastolic pump duration (68), a drive wheel deceleration, a drive wheel acceleration and/or an averaged pump flow.

3. Control and regulation unit (48) according to claim 1 or 2, further configured to determine the required value of the pulse parameter (20) from a given or predetermined mean arterial pressure (16) and/or a given or predetermined energy equivalent pressure (18), and/or further configured to determine the flow value (10) based on an inputted speed of the blood pump (40) or automatically based on a received flow measurement.

4. Control and regulation unit (48) according to any of the above claims, wherein the flow value (10) comprises a patient-specific mean flow (30), wherein the control and regulation unit (48) is further configured to adjust (22) the mean flow (30) based on a received flow measurement and the mean arterial pressure (16) by adjusting (22) the pulse parameter (20), and/or which is further configured to determine a heart rate from the ECG signal (14) and to adjust (22) the pulse parameter (20) in dependence of the heart rate.

5. Control and regulation unit (48) according to any of the preceding claims, which is further configured to determine an amplitude change from the ECG signal (14) and to adjust (22) the pulse parameter (20) at a predetermined time point after the amplitude change, preferably configured to determine an R-wave from the ECG signal (14) and to adjust the pulse parameter (20) at a predetermined time point after the R-wave (22).

6. Control and regulation unit (48) according to any of the preceding claims, which is further configured to receive a measurement of an aortic pressure of the supported patient (38) over a predetermined period of time and to adjust (22) the pulse parameter (20) at a predetermined aortic pressure and change of aortic pressure.

7. Control and regulation unit (48) according to any of preceding claims 5 to 6, which is further configured to determine a diastolic phase and a systolic phase of the heart of the supported patient (38) from the ECG signal (14) and/or the aortic pressure and to adjust (22) at least the pulse duration (34) such that it ends before the systolic phase.

8. System (36) for extracorporeal circulatory support of a patient (38) comprising:

   - a venous patient access (42) and an arterial patient access (44),
   - a non-occlusive blood pump (40) fluidically connected to the venous patient access (42) and the arterial patient access (44) and configured to provide blood flow from the venous patient access (42) to the arterial patient access (44),
   - an interface (24) for receiving a measurement of an arterial pressure (12) and an ECG signal (14; 54) of the patient (38), and
   - a control and regulation unit (48) according to any of the preceding claims 1 to 7.

9. System (36) according to claim 8, further comprising a membrane oxygenator and/or a membrane fan, preferably an extracorporeally arranged membrane oxygenator.

10. System (36) according to claim 8 or 9, further comprising an ECG apparatus communicatively connected to the interface (24),
and/or
wherein the non-occlusive blood pump (40) and/or control and regulation unit (48), preferably both, are configured to be arranged extracorporeally.

11. System (36) according to any of preceding claims 8 to 10 further comprising at least one cannula for inserting the arterial patient access (44) into the patient (38) and one cannula for inserting the venous patient access (42) into the patient (38), wherein the cannula for inserting the arterial patient access (44) is preferably formed for insertion into the aortic region or into the *a. femoralis* and/or the cannula for inserting the venous patient access (42) is formed for insertion into the right atrium or into the *vena cava*.

12. Method for controlling/regulating a non-occlusive blood pump of an extracorporeal circulatory support, comprising the steps of:

  - receiving a flow value (10) of the extracorporeal circulatory support;
  - receiving a measurement of an arterial pressure (12) and an ECG signal (14) of a supported patient (38) over a predetermined period of time;
  - determining a mean arterial pressure (16) of the extracorporeal circulatory support or of the supported patient from the measurement of the arterial pressure (12) and an energy equivalent pressure (18) from the flow value (10) and the arterial pressure (12);
  - determining a required value of at least one pulse parameter (20) of the blood pump from said mean arterial pressure (16), the energy equivalent pressure (18), and the ECG signal (14) so as to effect a ratio of the energy equivalent pressure (18) to the mean arterial pressure (16) of greater than 1.0; and
  - adjusting (22) the pulse parameter (20) in dependence on the ECG signal (14).

13. Method according to claim 12, wherein an R-wave is determined from the ECG signal (14) and the pulse parameter (20) is adjusted (22) at a predetermined time point after the R-wave.

14. Method according to claim 12 or 13, wherein the at least one pulse parameter (20) is selected from a pulse amplitude (32), a pump speed, a pump speed change per unit time, a pulse duration (34), a systolic pump duration (66), a diastolic pump duration (68), a drive wheel deceleration, a drive wheel acceleration, and/or an averaged pump flow,
and/or

wherein a diastolic phase and systolic phase of the heart of the supported patient (38) are determined from the ECG signal (14) and at least the pulse duration (32) is adjusted (22) such that it ends before the systolic phase,
and/or
wherein the required value of the pulse parameter (20) is further determined in dependence of a predetermined target value or a predetermined target percentage of the energy equivalent pressure (18).

15. Method according to any of preceding claims 12 to 14, wherein the non-occlusive blood pump (40) is fluidically connected to a membrane oxygenator.

16. Method according to any of preceding claims 12 to 15, wherein the method steps, as defined by claim 12, are carried out non-invasively.

17. Method according to any of preceding claims 12 to 16, wherein the method is carried out with a control and regulation unit (48) according to any of claims 1 to 7.


**Revendications**

1. Unité de commande et de réglage (48) pour une pompe à sang non occlusive (40) d'une assistance circulatoire extracorporelle, qui est établie pour

  - recevoir une valeur de flux (10) de l'assistance circulatoire extracorporelle ;
  - recevoir une mesure d'une pression artérielle (12) et d'un signal d'ECG (14) d'un patient assisté (38) pendant un temps prédéfini ;
  - déterminer une pression artérielle moyenne (16) de l'assistance circulatoire extracorporelle ou du patient assisté à l'aide de la mesure de la pression artérielle (12) et une pression équivalente d'énergie (18) à l'aide de la valeur de flux (10) et de la pression artérielle (16) ; et
  - déterminer une valeur nécessaire d'au moins un paramètre de pouls (20) de la pompe à sang (40) à l'aide de la pression artérielle moyenne (16), de la pression équivalente d'énergie (18) et du signal d'ECG (14) pour obtenir un rapport de la pression équivalente d'énergie (18) sur la pression artérielle moyenne (16) qui est supérieur à 1,0,

dans laquelle l'unité de commande et de réglage (48) est en outre établie pour régler le paramètre de pouls (20) en fonction du signal d'ECG (14).

2. Unité de commande et de réglage (48) selon la revendication 1, dans laquelle l'au moins un paramètre de pouls (20) est sélectionné à partir d'une amplitude de pouls (32), d'un régime de pompe, d'une modification de régime de pompe par unité de temps, d'une durée de pouls (34), d'une durée de pompe systolique (66), d'une durée de pompe diastolique (68), d'un ralentissement de roue d'entraînement, d'une accélération de roue d'entraînement et/ou d'un flux de pompe moyenné.

3. Unité de commande et de réglage (48) selon la revendication 1 ou 2, qui est en outre établie pour déterminer la valeur nécessaire du paramètre de pouls (20) à l'aide d'une pression artérielle moyenne prédéfinie (16) et/ou d'une pression équivalente d'énergie prédéfinie (18)
et/ou
qui est en outre établie pour déterminer la valeur de flux (10) à l'aide d'un régime entré de la pompe à sang (40) ou automatiquement à l'aide d'une mesure de flux reçue.

4. Unité de commande et de réglage (48) selon l'une quelconque des revendications précédentes, dans laquelle la valeur de flux (10) comprend un flux moyen spécifique au patient (30), dans laquelle l'unité de commande et de réglage (48) est en outre établie pour régler (22) le flux moyen (30) à l'aide d'une mesure de flux reçue et de la pression artérielle moyenne (16) par le biais d'un réglage (22) du paramètre de pouls (20)
et/ou
qui est en outre établie pour déterminer à partir du signal d'ECG (14) une fréquence cardiaque et régler (22) le paramètre de pouls (20) en fonction de la fréquence cardiaque.

5. Unité de commande et de réglage (48) selon l'une quelconque des revendications précédentes, qui est en outre établie pour déterminer une modification d'amplitude à partir du signal d'ECG (14) et régler (22) le paramètre de pouls (20) à un moment prédéfini après la modification d'amplitude,
qui est de préférence établie pour déterminer une onde R à partir du signal d'ECG (14) et régler le paramètre d'impulsion (20) à un moment prédéfini après l'onde R.

6. Unité de commande et de réglage (48) selon l'une quelconque des revendications précédentes, qui est en outre établie pour recevoir une mesure d'une pression de l'aorte du patient assisté (38) pendant un temps prédéfini et régler (22) le paramètre de pouls (20) lors d'une pression de l'aorte prédéfinie et d'une modification de la pression de l'aorte.

7. Unité de commande et de réglage (48) selon l'une quelconque des revendications précédentes 5 à 6, qui est en outre établie pour déterminer une phase diastolique et une phase systolique du cœur du patient assisté (38) à partir du signal d'ECG (14) et/ou la pression de l'aorte et régler au moins la durée de pouls (34) de telle sorte que celle-ci se termine avant la phase systolique.

8. Système (36) pour l'assistance circulatoire extracorporelle d'un patient (38), comprenant :

   - un accès aux veines du patient (42) et un accès aux artères du patient (44),
   - une pompe à sang non occlusive (40) qui est reliée de manière fluidique à l'accès aux veines du patient (42) et à l'accès aux artères du patient (44) et est conçue pour la mise à disposition d'un flux de sang de l'accès aux veines du patient (42) à l'accès aux artères du patient (44),
   - une interface (24) pour la réception d'une mesure d'une pression artérielle (12) et d'un signal d'ECG (14 ; 54) du patient (38), et
   - une unité de commande et de réglage (48) selon l'une quelconque des revendications précédentes 1 à 7.

9. Système (36) selon la revendication 8, comprenant en outre un oxygénateur de membrane et/ou un ventilateur de membrane, de préférence un oxygénateur de membrane disposé de manière extracorporelle.

10. Système (36) selon la revendication 8 ou 9, comprenant en outre un appareil d'ECG qui est relié de manière à communiquer avec l'interface (24)
et/ou
dans lequel la pompe à sang non occlusive (40) et/ou l'unité de commande et de réglage (48), de préférence les deux sont conçues pour une disposition extracorporelle.

11. Système (36) selon l'une quelconque des revendications 8 à 10, comprenant en outre au moins une canule pour l'introduction de l'accès aux artères du patient (44) dans le patient (38) et une canule pour l'introduction de l'accès aux veines du patient (42) dans le patient (38), dans lequel la canule est configurée de préférence pour l'introduction de l'accès aux artères du patient (44) pour l'introduction dans la zone de l'aorte ou dans l'artère fémorale et/ou la canule pour l'introduction de l'accès aux veines du patient (42) est configurée pour l'introduction dans l'atrium droit ou dans la veine cave.

12. Procédé de commande/réglage d'une pompe à sang non occlusive d'une assistance circulatoire extracorporelle, comprenant les étapes de :

   - réception d'une valeur de flux (10) d'une assistance circulatoire extracorporelle ;

- réception d'une mesure d'une pression artérielle (12) et d'un signal d'ECG (14) d'un patient assisté (38) pendant un temps prédéfini ;

- détermination d'une pression artérielle moyenne (16) de l'assistance circulatoire extracorporelle ou du patient assisté à l'aide de la mesure de la pression artérielle (12) et d'une pression équivalente d'énergie (18) à l'aide de la valeur de flux (10) et de la pression artérielle (12) ;

- détermination d'une valeur nécessaire d'au moins un paramètre de pouls (20) de la pompe à sang à l'aide de la pression artérielle moyenne (16), de la pression équivalente d'énergie (18) et du signal d'ECG (14) pour obtenir un rapport de la pression équivalente d'énergie (18) sur la pression artérielle moyenne (16) qui est supérieur à 1,0 ; et

- réglage (22) du paramètre de pouls (20) en fonction du signal d'ECG (14).

13. Procédé selon la revendication 12, dans lequel une onde R est déterminée à partir du signal d'ECG (14) et le paramètre de pouls (20) est réglé à un moment prédéfini après l'onde R.

14. Procédé selon la revendication 12 ou 13, dans lequel l'au moins un paramètre de pouls (20) est sélectionné à partir d'une amplitude de pouls (32), d'un régime de pompe, d'une modification de régime de pompe par unité de temps, d'une durée de pouls (34), d'une durée de pompe systolique (66), d'une durée de pompe diastolique (68), d'un ralentissement de roue d'entraînement, d'une accélération de roue d'entraînement et/ou d'un flux de pompe moyenné
et/ou

dans lequel une phase diastolique et une phase systolique du cœur du patient assisté (38) sont déterminées à partir du signal d'ECG (14) et au moins la durée de pouls (32) est réglée (22) de telle sorte que celle-ci se termine avant la phase systolique
et/ou
dans lequel la valeur nécessaire du paramètre de pouls (20) est déterminée en outre en fonction d'une valeur cible prédéfinie ou d'une valeur de pourcentage cible prédéfinie de la pression équivalente d'énergie (18).

15. Procédé selon l'une quelconque des revendications précédentes 12 à 14, dans lequel la pompe à sang non occlusive (40) est reliée à un oxygénateur de membrane de manière fluidique.

16. Procédé selon l'une quelconque des revendications précédentes 12 à 15, dans lequel les étapes de procédé, comme le définit la revendication 12, sont menées à bien de manière non invasive.

17. Procédé selon l'une quelconque des revendications précédentes 12 à 16, dans lequel le procédé est mené à bien avec une unité de commande et de réglage (48) selon l'une quelconque des revendications 1 à 7.

Fig. 1

Fig. 2

Fig. 3

Fig. 4

Fig. 5

Fig. 6

Fig. 7A

Fig. 7B

Fig. 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20150141911 A1